# EUROPÄISCHE PATENTSCHRIFT

(11) **EP 0 842 146 B1**
(45) Veröffentlichungstag und Bekanntmachung des Hinweises auf die Patenterteilung: **23.08.2000**
(21) Anmeldenummer: 96924876.4
(22) Anmeldetag: 04.07.1996
(51) Int. Cl.: C07C 255/62, C07D 239/34, C07D 285/18, C07C 331/10, C07C 251/60, C07D 309/12, C07C 69/734, A01N 37/34, A01N 43/82, A01N 47/48, A01N 37/50

(54) **OXIMETHER- UND ACRYLSÄUREDERIVATE UND IHRE VERWENDUNG ALS FUNGIZIDE**
OXIMETHER AND ACRYLIC ACID DERIVATIVES AND THEIR USE AS FUNGICIDES
DERIVES D'ACIDE ACRYLIQUE ET D'OXYMETHER ET LEUR UTILISATION COMME FONGICIDES

(30) Priorität: 17.07.1995 DE 19525969
(43) Veröffentlichungstag der Anmeldung: 20.05.1998
(73) Patentinhaber: BAYER AG, 51368 Leverkusen (DE)
(72) Erfinder: GERDES, Peter, D-52080 Aachen (DE); GAYER, Herbert, D-40789 Monheim (DE); HEINEMANN, Ulrich, D-42799 Leichlingen (DE); TIEMANN, Ralf, D-51375 Leverkusen (DE); STENZEL, Klaus, D-40595 Düsseldorf (DE); DUTZMANN, Stefan, D-40721 Hilden (DE)
(86) Internationale Anmeldenummer: EP9602931
(87) Internationale Veröffentlichungsnummer: WO9703950

(56) Entgegenhaltungen:
- EP-A- 0 461 797
- DE-A- 4 424 788

## Beschreibung

Die Erfindung betrifft neue Etherderivate, mehrere Verfahren zu ihrer Herstellung und ihre Verwendung als Fungizide, sowie neue Zwischenprodukte und mehrere Verfahren zu deren Herstellung.

Es ist bereits bekannt geworden, daß bestimmte Methoximinoessigsäure- sowie Methoxyacrylsäurederivate, die den unten beschriebenen Etherderivaten konstitutionell ähnlich sind, fungizide Eigenschaften besitzen (vergleiche z. B. EP-A-226917 oder EP-A-370629 oder EP-A-398692). In DE-A-4424788 werden weitere Methoximinoessigsäure- sowie Methoxyacrylsäurederivate mit fungiziden Eigenschaften beschrieben. Die fungizide Wirkung dieser Verbindungen ist jedoch in vielen Fällen unbefriedigend.

Es wurden nun die neuen Etherderivate der allgemeinen Formel (I) gefunden, 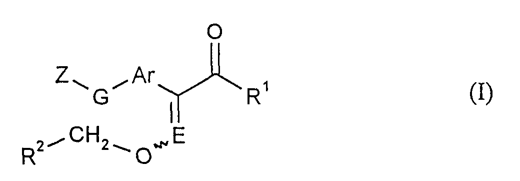 in welcher
- Ar: für jeweils gegebenenfalls substituiertes Arylen oder Heteroarylen steht,
- E: für =CH- oder Stickstoff steht,
- G: für eine Einfachbindung, für Sauerstoff, Schwefel oder für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen -Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-NOC(R³), -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-O-CH₂-, -C(=N-O-R⁵)-C(R³)-O-N=CH-, -C(=N-O-R⁵)-C(R³)-O-N=C(CH₃)-, -T-Ar¹- oder -T-Ar¹-Q- steht, wobei
Ar¹ für gegebenenfalls substituiertes Arylen, Heteroarylen, Cycloalkylen oder Heterocycloalkylen (d. h. ein zweifach verknüpfter aliphatischer Ring, in dem ein oder mehrere Kohlenstoffatome durch Heteroatome, d.h. von Kohlenstoff verschiedene Atome ersetzt sind) steht,
n für die Zahlen 0, 1 oder 2 steht,
Q für Sauerstoff oder Schwefel steht,
R³ fürWasserstoff, Cyano oder jeweils gegebenenfalls substituiertes Alkyl, Alkoxy, Alkylthio, Alkylamino, Dialkylamino oder Cycloalkyl steht, und
R⁴ für Wasserstoff, Hydroxy, Cyano oder jeweils gegebenenfalls substituiertes Alkyl, Alkoxy oder Cycloalkyl steht, und
R⁵ für Wasserstoff oder Alkyl steht, und
T für eine Einfachbindung, für Sauerstoff, Schwefel, -CH₂-O-, -CH₂-S- oder für gegebenenfalls substituiertes Alkandiyl steht,
- R¹: für Alkoxy, Alkylamino oder Dialkylamino steht,
- R²: für Cyano, Thiocyanato, Alkylcarbonyl, Alkoxycarbonyl, Aminocarbonyl, Aminothicarbonyl, Alkylaminocarbonyl oder Dialkylaminocarbonyl steht, und
- Z: für jeweils gegebenenfalls substituiertes Alkyl, Alkenyl, Alkinyl, Cycloalkyl, Aryl oder Heterocyclyl steht.

In den Definitionen sind die gesättigten oder ungesättigten Kohlenwasserstoffketten, wie Alkyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono oder polycyclische Kohlenwasserstoffringe, beispielhaft und vorzugsweise sind Phenyl, Naphthyl, Anthranyl, Phenanthryl, vorzugsweise Phenyl oder Naphthyl, insbesondere Phenyl genannt.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Weiterhin wurde gefunden, daß man die neuen Etherderivate der allgemeinen Formel (I) erhält, wenn man
a) Hydroxyverbindungen der allgemeinen Formel (II) 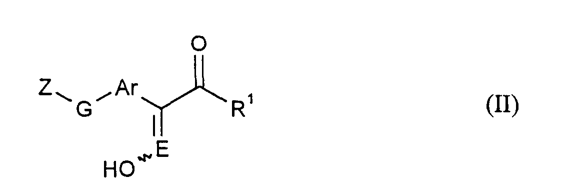 in welcher
   Ar, E, G, R¹ und Z die oben angegebenen Bedeutungen haben,
   mit einer Halogenverbindung der allgemeinen Formel (III)

   X¹-CH₂-R² (III)

   in welcher
   - X¹: für Halogen steht und
   - R²: die oben angegebene Bedeutung hat,
   gegebenenfalls in Gegenwart eines Säureakzeptors und gegebenenfalls in Gegenwart eines Verdünnungsmittels umsetzt; oder wenn man
b) Hydroxyarylverbindungen der allgemeinen Formel (IV) 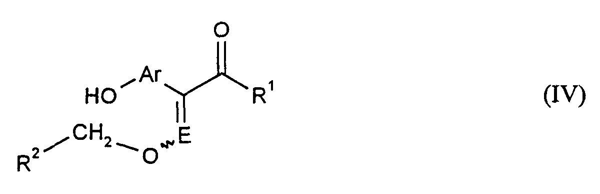 in welcher
   - Ar, E, R¹ und R²: die oben angegebenen Bedeutungen haben,
   mit einer Arylverbindung der allgemeinen Formel (V) 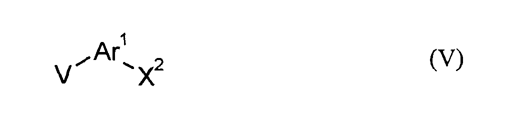 in welcher
   - Ar¹: die oben angegebene Bedeutung hat,
   - V: für Halogen oder Z-T- steht, wobei
   - Z und T: die oben angegebenen Bedeutungen haben und
   - X²: für Halogen, Alkylsulfonyl oder Arylsulfonyl steht,
   gegebenenfalls in Gegenwart eines Säureakzeptors und gegebenenfalls in Gegenwart eines Verdünnungsmittels umsetzt.

Schließlich wurde gefunden, daß die neuen Etherderivate der allgemeinen Formel (I) sehr starke fungizide Wirkung zeigen.

Die erfindungsgemäßen Verbindungen können gegebenenfalls als Mischungen verschiedener möglicher isomerer Formen, insbesondere von Stereoisomeren, wie z. B. E- und Z-, vorliegen. Es werden sowohl die E- als auch die Z-Isomeren, wie auch beliebige Mischungen dieser Isomeren, beansprucht.

Die erfindungsgemäßen Etherderivate sind durch die Formel (I) allgemein definiert. In dieser Formel sind die einzelnen Definitionen vorzugsweise und jeweils unabhängig voneinander definiert, wobei:
- Ar: steht vorzugsweise für jeweils gegebenenfalls substituiertes Phenylen oder Naphthylen, für mono- oder bicyclisches Heteroarylen mit jeweils 5 oder 6 Ringgliedern oder für benzokondensiertes Heteroarylen mit 5 oder 6 Ringgliedern, von denen jeweils mindestens eines für Sauerstoff, Schwefel oder Stickstoff steht und gegebenenfalls ein oder zwei weitere für Stickstoff stehen, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen.
- E: steht vorzugsweise für =CH- oder Stickstoff.
- G: steht vorzugsweise für eine Einfachbindung, für Sauerstoff, Schwefel oder für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-O-CH₂-, -C(=N-O-R⁵)-C(R³)-O-N=CH-, -C(=N-O-R⁵)-C(R³)-O-N=C(CH₃)-, -T-Ar¹- oder -T-Ar¹-Q-, wobei
n für die Zahlen 0, 1 oder 2 steht,
Q für Sauerstoff oder Schwefel steht,
R³ für Wasserstoff, Cyano, für jeweils gegebenenfalls durch Halogen, Cyano oder C₁-C₁-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₁-Alkyl oder C₁-C₁-Alkoxy-carbonyl substituiertes Cycloalkyl mit 3 bis 6 Kohlenstoffatomen steht, und
R⁴ für Wasserstoff, Hydroxy, Cyano oder für gegebenenfalls durch Halogen, Cyano oder C₁-C₁-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht,
R⁵ für Wasserstoff oder Alkyl mit 1 bis 4 Kohlenstoffatomen steht,
Ar¹ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie;
Cycloalkyl mit 3 bis 6 Kohlenstoffatomen und
T für eine Einfachbindung, für Sauerstoff, Schwefel, -CH₂-O-, -CH₂-S- oder für Alkandiyl mit 1 bis 3 Kohlenstoffatomen steht.
- R¹: steht vorzugsweise für Alkyl, Alkoxy, Alkylamino oder Dialkylamino mit 1 bis 4 Kohlenstoffatomen in den jeweiligen Alkylketten.
- R²: steht vorzugsweise für Cyano, Thiocyanato, Aminocarbonyl, Aminothiocarbonyl oder für Alkylcarbonyl, Alkoxycarbonyl, Alkylaminocarbonyl oder Dialkylaminocarbonyl mit 1 bis 4 Kohlenstoffatomen in den jeweiligen Alkylketten.
- Z: steht vorzugsweise für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen;
für jeweils gegebenenfalls einfach oder mehrfach, gleich oder verschieden substituiertes Phenyl, Naphthyl oder für Heterocyclyl mit 3 bis 7 Ringgliedern, von denen mindestens eines für Sauerstoff, Schwefel oder Stickstoff und gegebenenfalls ein oder zwei weitere für Stickstoff stehen, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -
oder eine Gruppierung 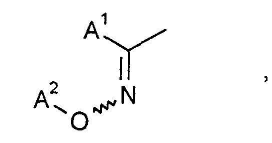 worin
- A¹: für Alkyl mit 1 bis 4 Kohlenstoffatomen oder Cycloalkyl mit 1 bis 6 Kohlenstoffatomen steht und
- A²: für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht.
- Ar: steht insbesondere für jeweils gegebenenfalls einfach oder zweifach durch Fluor, Chlor, Cyano, Methyl, Ethyl, Cyclopropyl, Trifluormethyl, Methoxy, Ethoxy, Methylthio, Methylsulfinyl oder Methylsulfonyl substituiertes ortho-, meta- oder para-Phenylen, Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl.
- E: steht insbesondere für =CH- oder Stickstoff.
- G: steht insbesondere für Sauerstoff oder für jeweils gegebenenfalls durch Fluor, Chlor oder Brom substituiertes Dimethylen (Ethan-1,2-diyl), Ethen-1,2-diyl oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³=N-O-CH₂-, -C(=N-O-R⁵)-C(R³-C(R³)=N-O-CH₂-, -C(=N-O-R⁵)-C(R³)-O-N=CH-, -C(=N-O-R⁵)-C(R³)-O-N=C(CH₃)-, -T-Ar¹- oder -T-Ar¹-Q-, wobei
n für die Zahlen 0, 1 oder 2 steht,
Q für Sauerstoff oder Schwefel steht,
R³ für Wasserstoff, Cyano, Methyl, Ethyl oder Cyclopropyl steht und
R⁴ für Wasserstoff, Methyl, Ethyl oder Cyclopropyl steht,
R⁵ für Wasserstoff oder Methyl steht,
Ar¹ für jeweils gegebenenfalls einfach bis dreifach, gleich oder verschieden substituiertes Phenylen oder Pyridindiyl, für jeweils gegebenenfalls einfach substituiertes Pyrimidindiyl, Pyridazindiyl, Pyrazindiyl, 1,2,3-Triazindiyl, 1,2,4-Triazindiyl oder 1,3,5-Triazindiyl oder für 1,2,4-Thiadiazoldiyl, 1,3,4-Thiadiazoldiyl, 1,2,4-Oxadiazoldiyl, 1,3,4-Oxadiazoldiyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
T für eine Einfachbindung, für Sauerstoff, Schwefel, -CH₂-O-, CH₂-S-, Methylen, Ethylen oder Propylen steht.
- R¹: steht insbesondere für Methyl, Ethyl, n- oder i-Propyl, Methoxy, Ethoxy, n- oder i-Propoxy-, Methylamino, Ethylamino, n- oder i-Propylamino, Dimethylamino, Diethylamino oder N-Methyl-N-ethylamino.
- R²: steht insbesondere für Cyano, Thiocyanato, Aminocarbonyl, Aminothiocarbonyl, Acetyl, Propionyl, Pivaloyl, Methoxycarbonyl, Ethoxycarbonyl, Methylaminocarbonyl, Ethylaminocarbonyl oder Dimethylaminocarbonyl.
- Z: steht insbesondere für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
oder eine Gruppierung 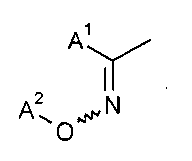
A¹ steht insbesondere für Methyl, Ethyl, n- oder i-Propyl, n-, i-, s- oder t-Butyl, Cyclopropyl oder Cyclobutyl.
A² steht insbesondere für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl.

Eine besonders bevorzugte Gruppe erfindungsgemäßer Verbindungen sind diejenigen Verbindungen der Formel (I),
in welcher
- Ar: für ortho-Phenylen, Pyridin-2,3-diyl oder Thiophen-2,3-diyl steht,
- E: für =CH- oder Stickstoff steht,
- G: für -O-CH₂- steht,
- R¹: für Methoxy oder Methylamino steht,
- R²: für Cyano, Thiocyanato, Aminocarbonyl, Aminothiacarbonyl, Acetyl, Propionyl, Pivaloyl, Methoxycarbonyl, Ethoxycarbonyl, Methylaminocarbonyl, Ethylaminocarbonyl oder Dimethylaminocarbonyl steht, und
- Z: für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy
oder eine Gruppierung 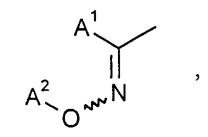 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

Eine ebenfalls besonders bevorzugte Gruppe erfindungsgemäßer Verbindungen sind diejenigen Verbindungen der Formel (I),
in welcher
- Ar: für ortho-Phenylen, Pyridin-2,3-diyl oder Thiophen-2,3-diyl steht,
- E: für =CH- oder Stickstoff steht,
- G: für -C(R³)=N-O-CH₂- steht,
- R³: für Methyl oder Cyclopropyl steht,
- R¹: für Methoxy oder Methylamino steht,
- R²: für Cyano, Thiocyanato, Aminocarbonyl, Aminothiocarbonyl, Acetyl, Propionyl, Pivaloyl, Methoxycarbonyl, Ethoxycarbonyl, Methyl aminocarbonyl, Ethylaminocarbonyl oder Dimethylaminocarbonyl steht, und
- Z: für jeweils gegebenenfalls einfach bis dreifach, gleich oder verschieden substituiertes Phenyl, Pyridyl oder Pyrimidyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy.

Eine weiterhin besonders bevorzugte Gruppe erfindungsgemäßer Verbindungen sind diejenigen Verbindungen der Formel (I),
in welcher
- Ar: für ortho-Phenylen steht,
- E: für =CH- oder Stickstoff steht,
- G: für Sauerstoff oder -T-Ar¹-O- steht,
- Ar¹: für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
- R¹: für Methoxy oder Methylamino steht,
- R²: für Cyano, Thiocyanato, Aminocarbonyl, Aminothiocarbonyl, Acetyl, Propionyl, Pivaloyl, Methoxycarbonyl, Ethoxycarbonyl, Methyl aminocarbonyl, Ethylaminocarbonyl oder Dimethylaminocarbonyl steht,
- T: für eine Einfachbindung, für Sauerstoff, Schwefel, -CH₂-O-, CH₂-S-, Methylen, Ethylen oder Propylen steht und
- Z: für jeweils gegebenenfalls einfach bis dreifach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy, Difluorchlormethoxy, Trifluorethoxy, Trifluormethoxy oder jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy, substituiertes Phenyl, Pyridyl, Pyrimidyl oder Thienyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Beispiele für die erfindungsgemäßen Verbindungen sind in den Tabellen 1 bis 16 aufgeführt:

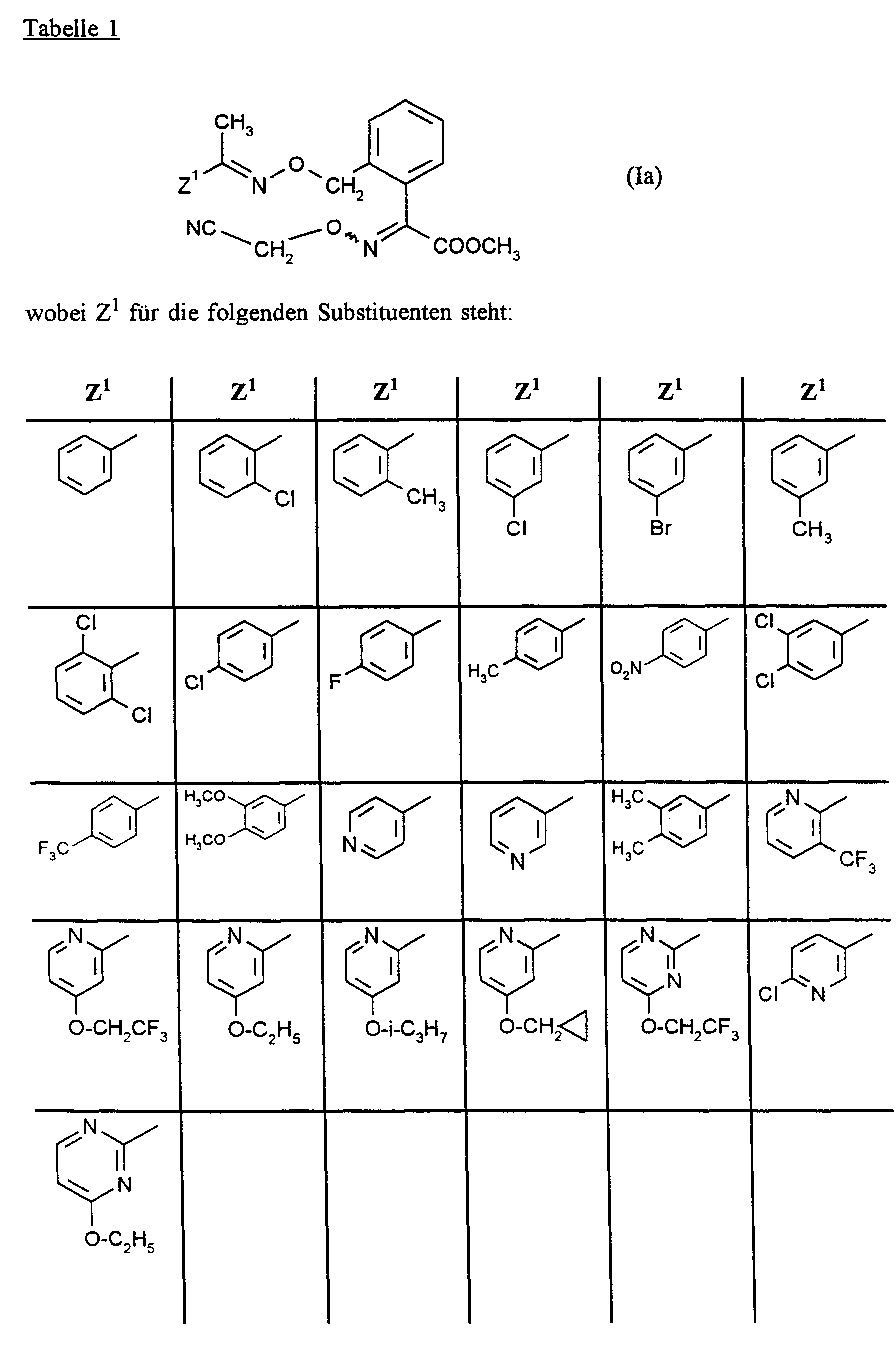

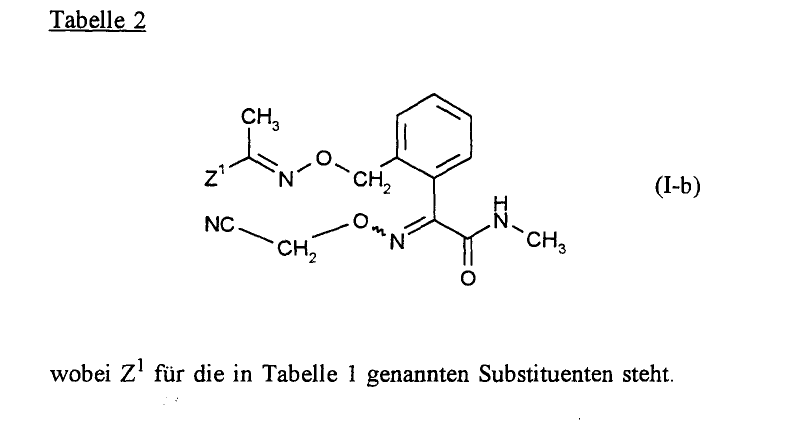

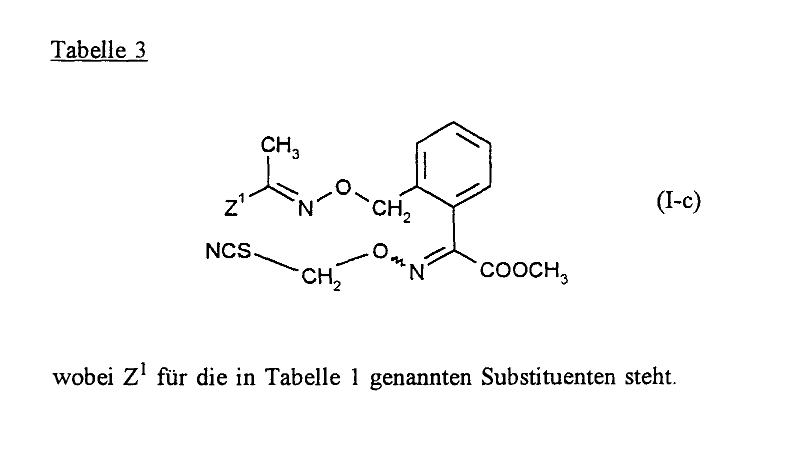

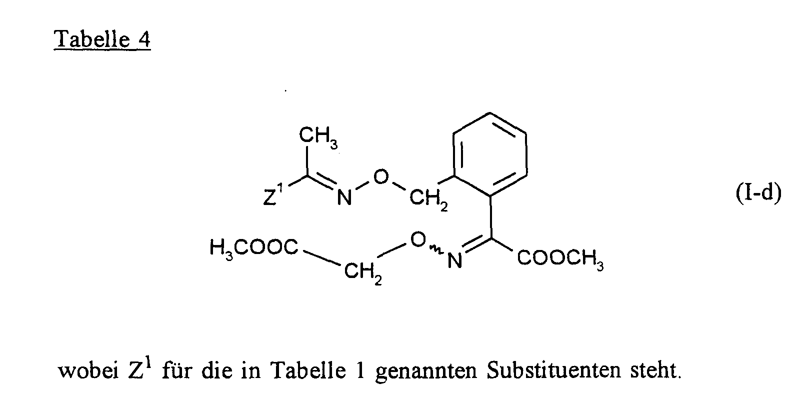

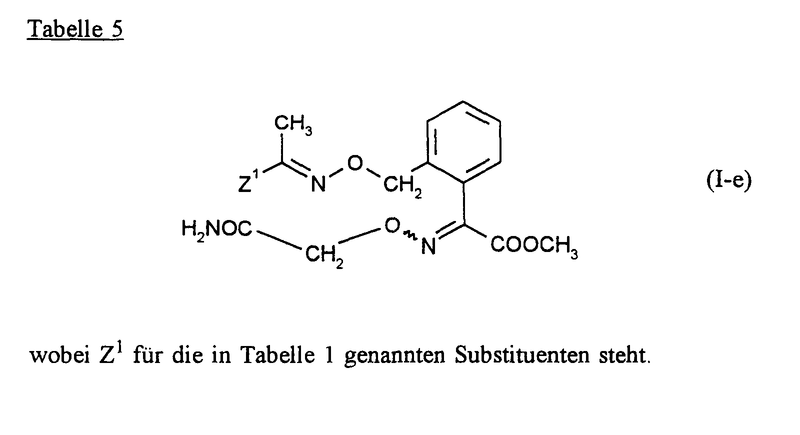

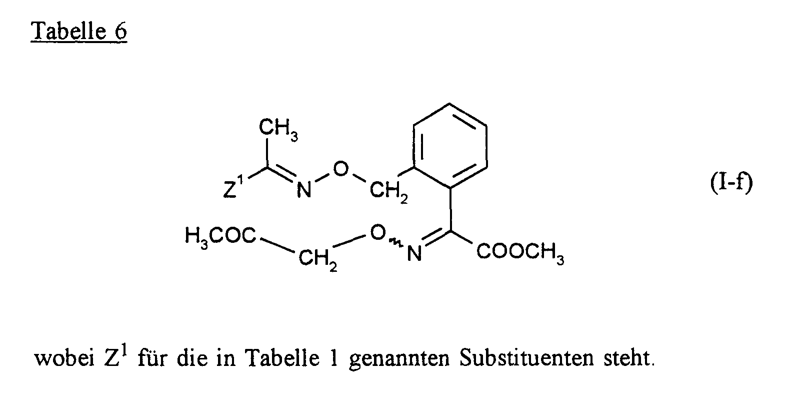

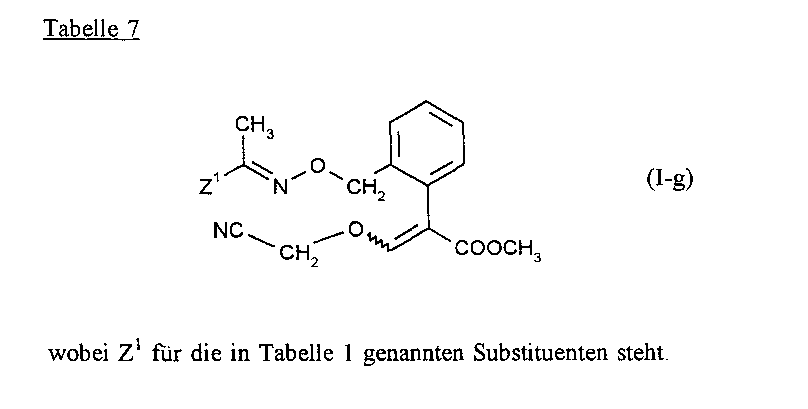

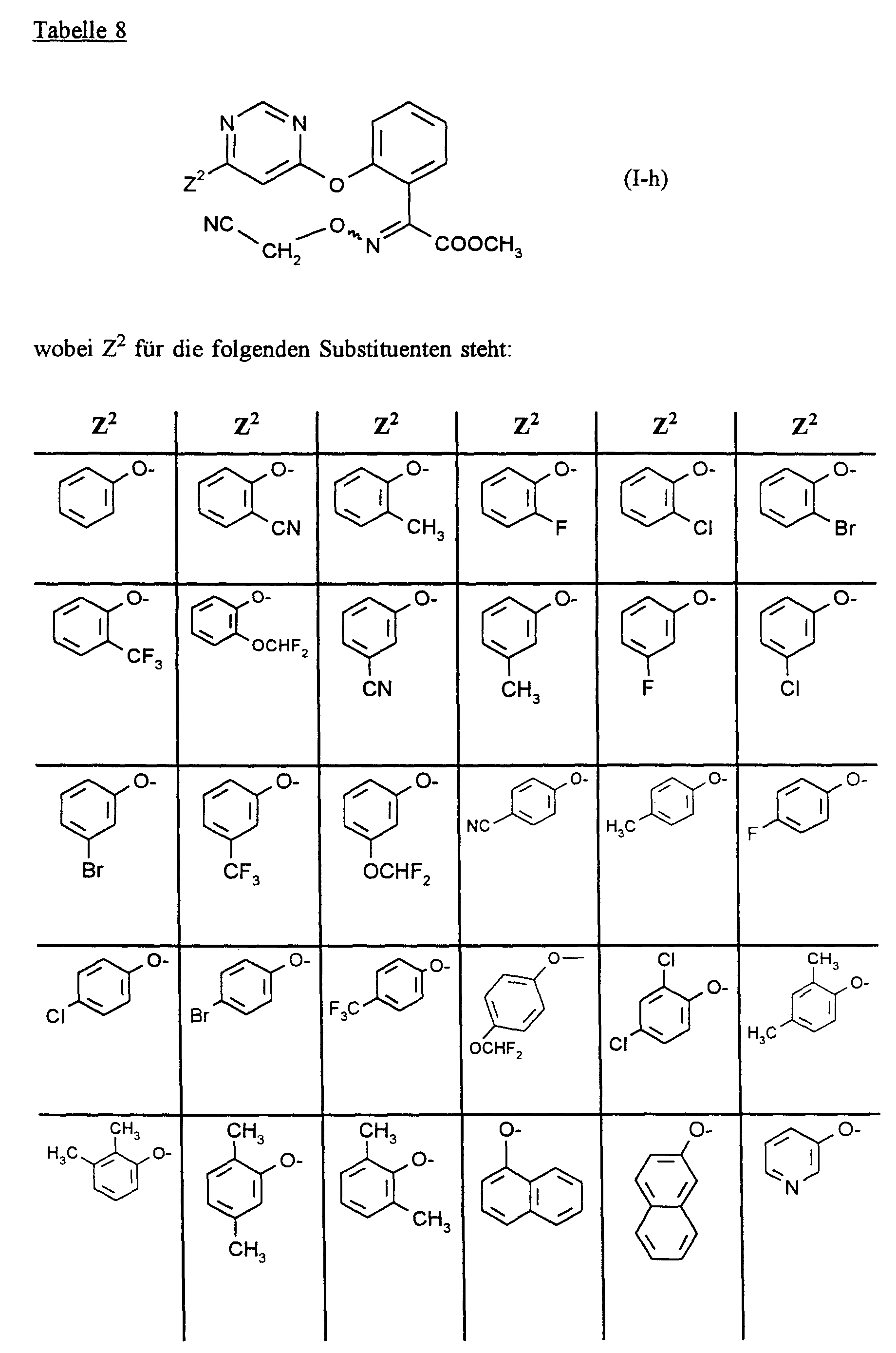

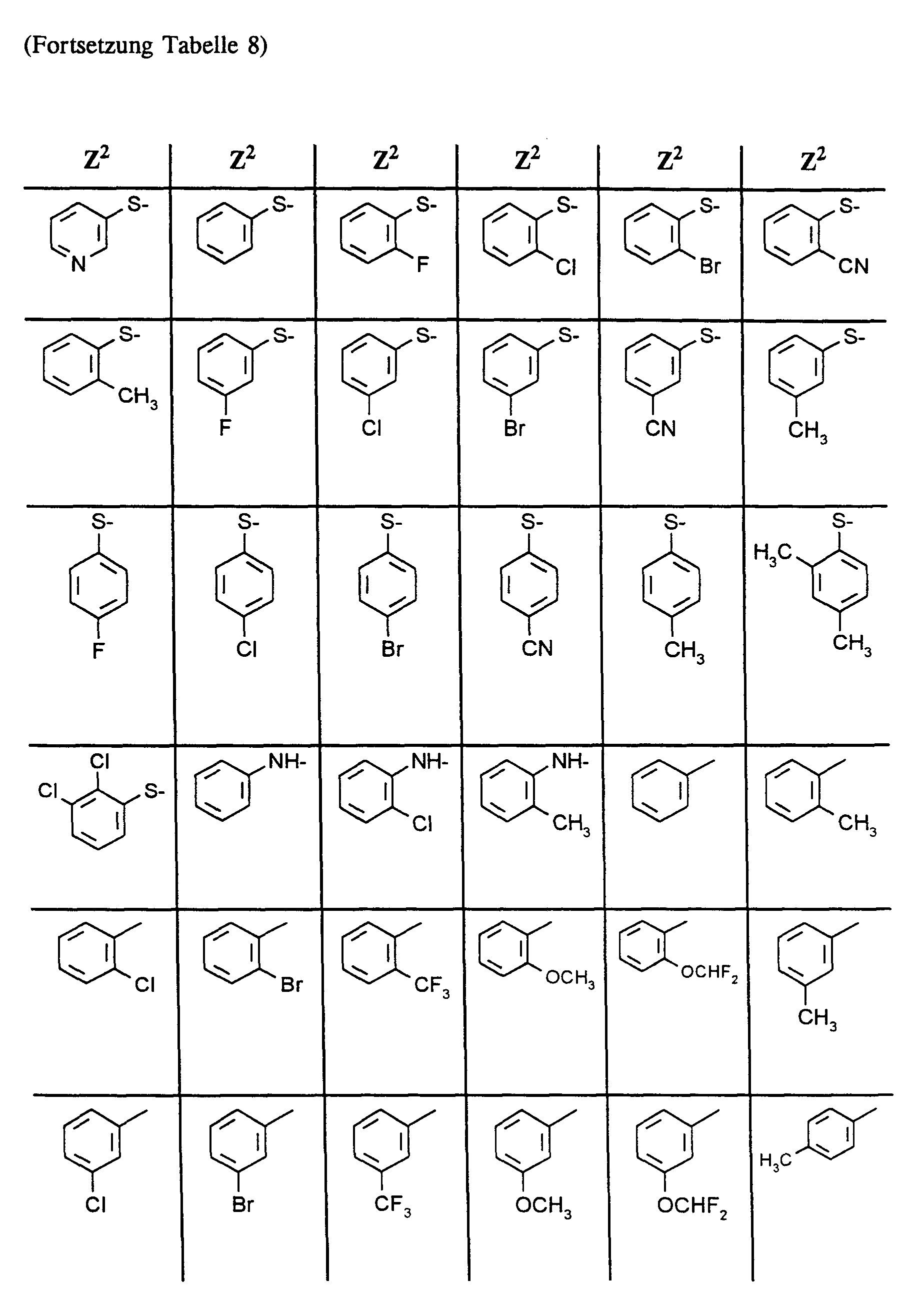

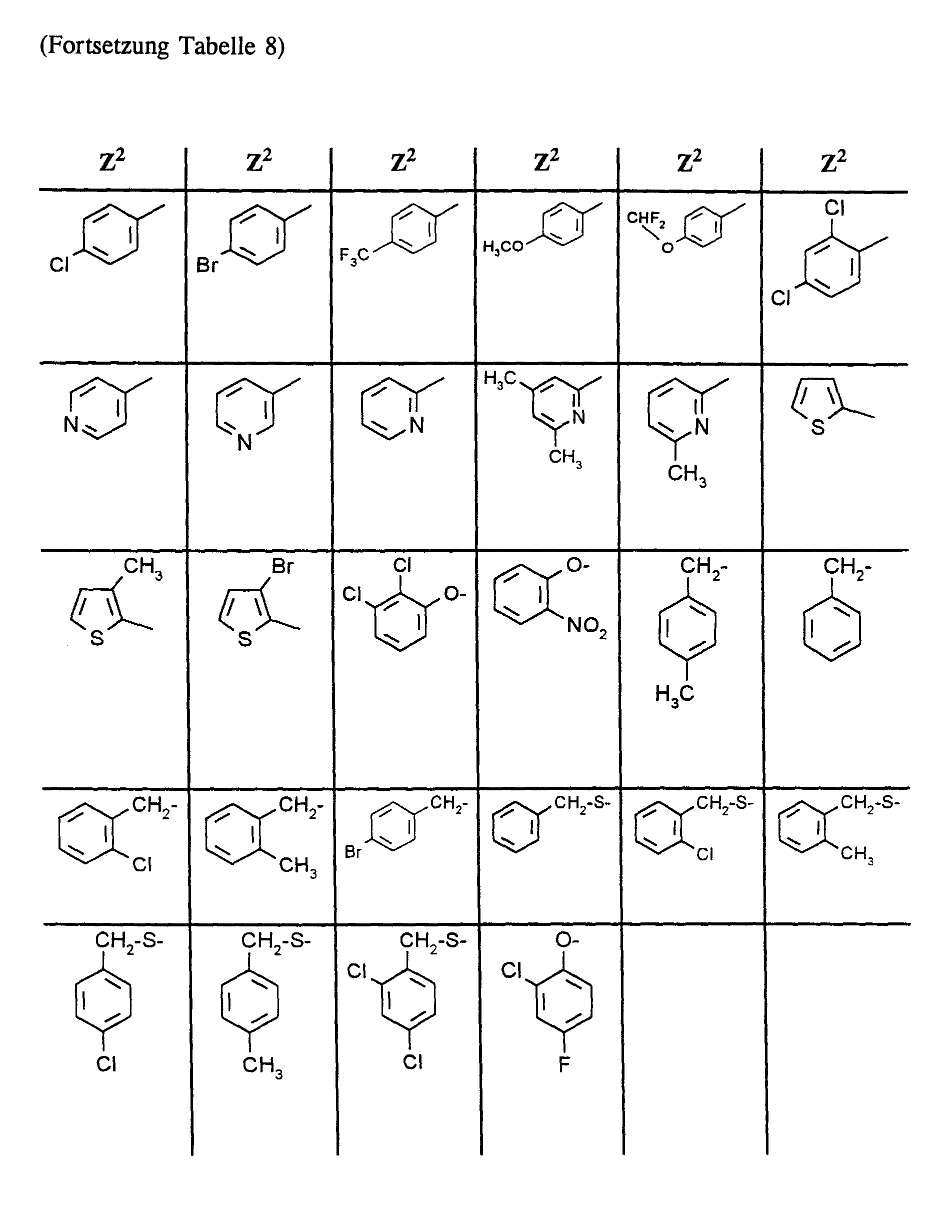

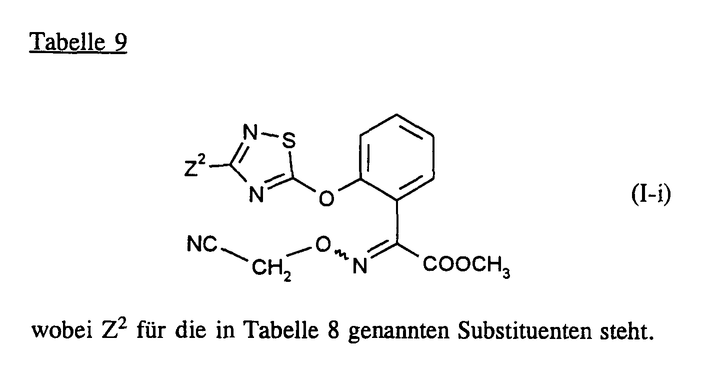

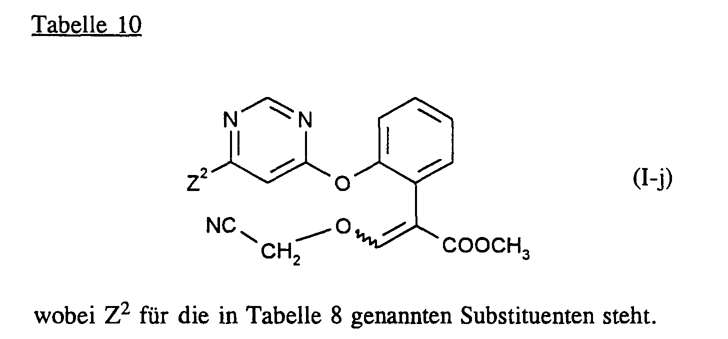

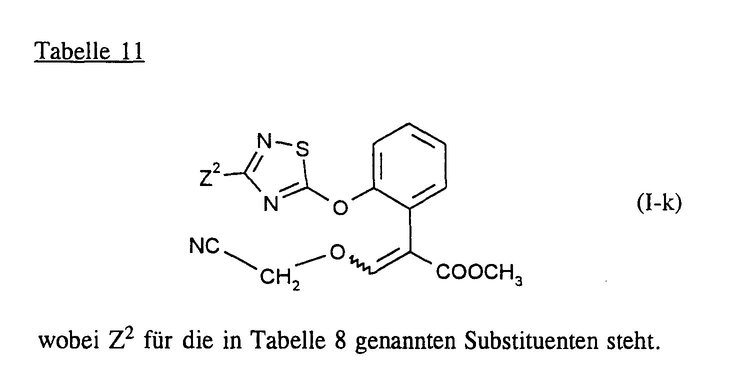

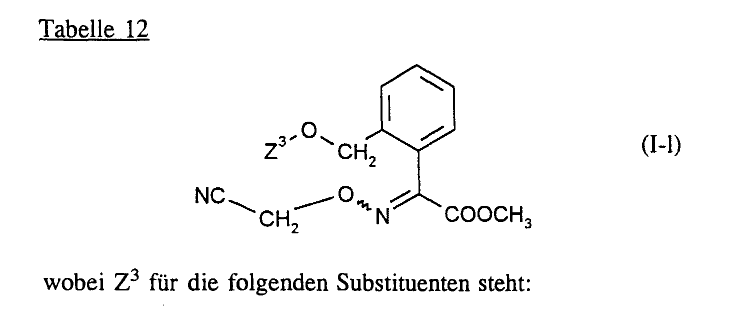

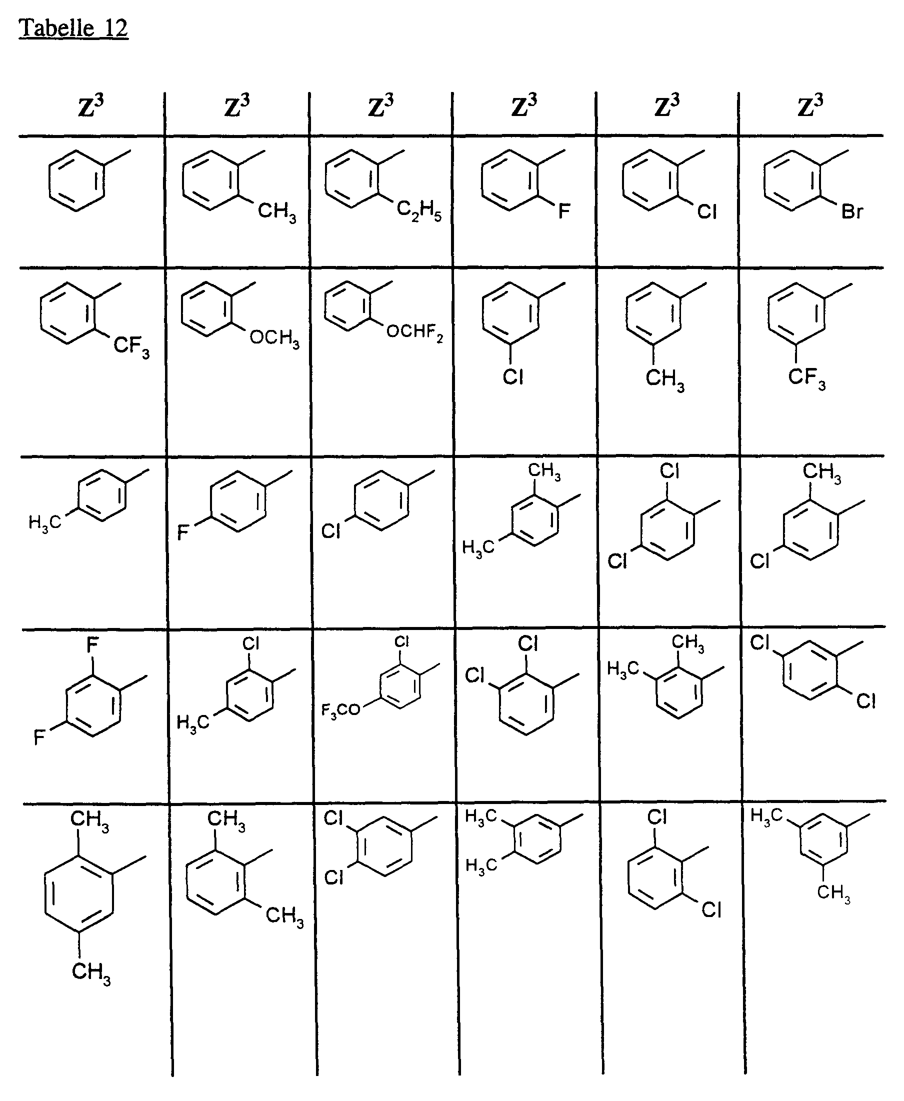

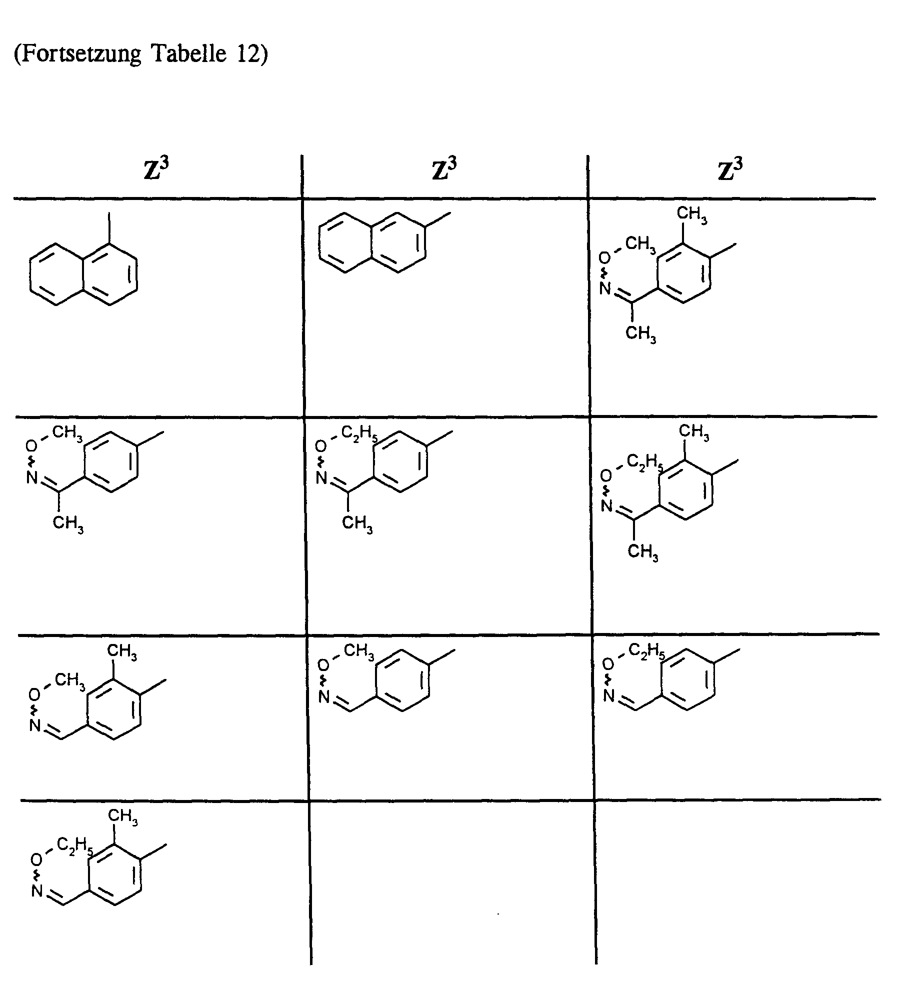

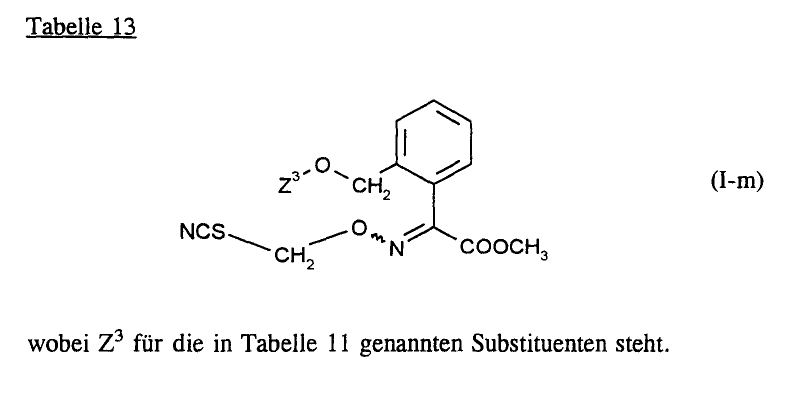

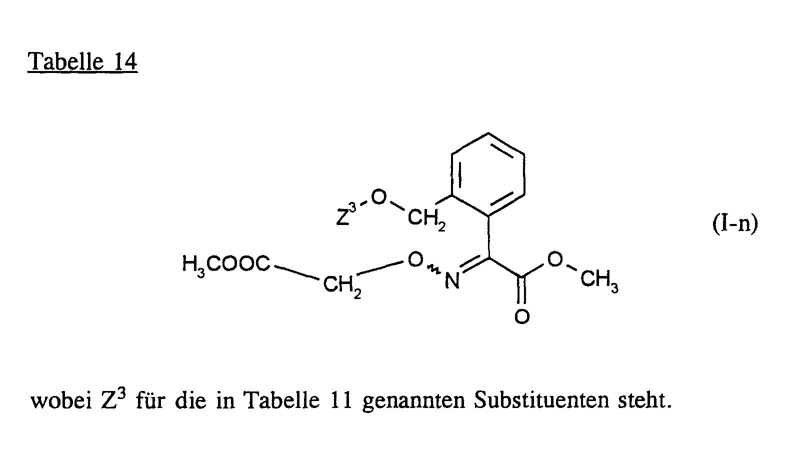

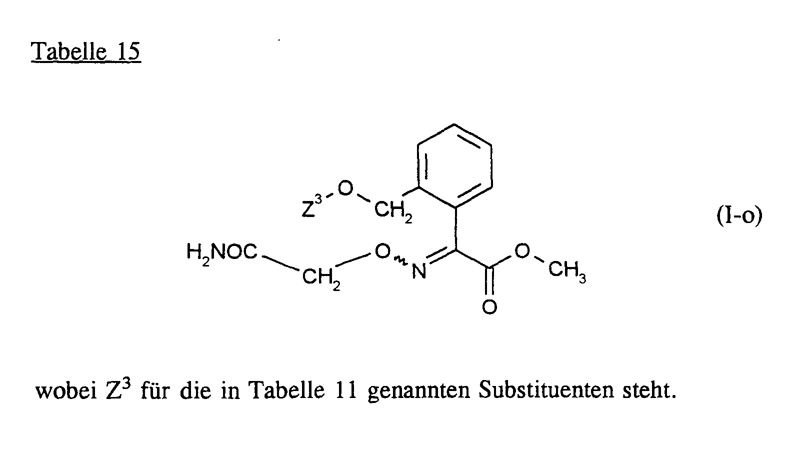

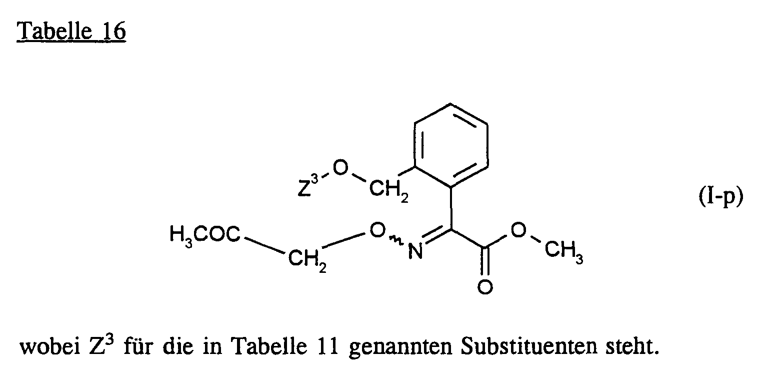

Die zur Durchführung des erfindungsgemäßen Verfahrens a) als Ausgangsstoffe benötigten Hydroxyverbindungen sind durch die Formel (II) allgemein definiert. In dieser Formel (II) haben Ar, E, G, R¹ und Z vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Ar, E, G, R¹ und Z angegeben wurden.

Die Ausgangsstoffe der Formel (II) sind bekannt und/oder können nach an sich bekannten Verfahren hergestellt werden (vgl. z. B. EP-A 554767).

Die zur Durchführung des erfindungsgemäßen Verfahrens a) weiterhin als Ausgangsstoffe benötigten Halogenverbindungen sind durch die Formel (III) allgemein definiert. In der Formel (III) hat R² vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R² angegeben wurde. X¹ steht für Halogen, vorzugsweise für Chlor oder Brom.

Die Halogenverbindungen der allgemeinen Formel (III) sind bekannte Reagenzien in der organischen Chemie.

Die zur Durchführung des erfindungsgemäßen Verfahrens b) als Ausgangsstoffe benötigten Hydroxyarylverbindungen sind durch die Formel (IV) allgemein definiert. In dieser Formel (IV) haben Ar, E, R¹ und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Ar, E, R¹ und R² angegeben wurden.

Die Hydroxyarylverbindungen der Formel (IV) sind noch nicht bekannt; sie sind als neue Stoffe Gegenstand der vorliegenden Anmeldung.

Die Hydroxyarylverbindungen der Formel (IV) werden erhalten (Verfahren c), wenn man Tetrahydropyranylether der Formel (VI), 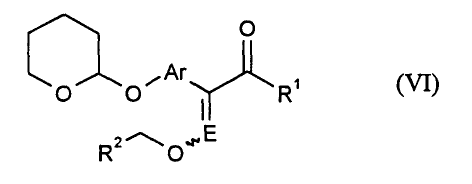 in welcher
- Ar, E, R¹ und R²: die oben angegebenen Bedeutungen haben,
gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Ketons, wie Aceton, Butanon, Methylisobutylketon oder Cyclohexanon; eines Nitrils, wie Acetonitril, Propionitril, n- oder i-Butyronitril oder Benzonitril; eines Esters wie Essigsäuremethylester oder Essigsäureethylester; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder reinem Wasser und gegebenenfalls in Gegenwart einer Säure, vorzugsweise einer anorganischen oder organischen Protonen- oder Lewissäure, wie beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, oder auch einer polymeren Säure wie beispielsweise einem sauren Ionenaustauscher, einer sauren Tonerde oder saurem Kieselgel, bei Temperaturen von -20°C bis 120°C, vorzugsweise bei Temperaturen von -10°C bis 80°C, hydrolysiert.

Die zur Durchführung des erfindungsgemäßen Verfahrens c) als Ausgangsstoffe benötigten Tetrahydropyranylether sind durch die Formel (VI) allgemein definiert. In dieser Formel (VI) haben Ar, E, R¹ und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Ar, E, R¹ und R² angegeben wurden.

Die Tetrahydropyranylether der Formel (VI) sind noch nicht bekannt; sie sind als neue Stoffe Gegenstand der vorliegenden Anmeldung.

Die Tetrahydropyranylether der Formel (VI) werden erhalten, wenn man (Verfahren dl) Arylessigsäurederivate der Formel (VII), 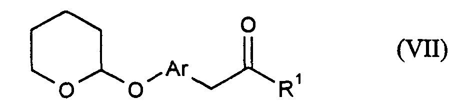 in welcher
- Ar und R¹: die oben angegebenen Bedeutungen haben,
zunächst mit einem Ameisensäurederivat, wie beispielsweise Ameisensäuremethylester, Kohlenmonoxid, eines Dialkylformamidacetals oder eines Bis-dialkylaminoalkoxymethans, gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Ketons, wie Aceton, Butanon, Methyl-isobutylketon oder Cyclohexanon; eines Nitrils, wie Acetonitril, Propionitril, n- oder i-Butyronitril oder Benzonitril; eines Esters wie Essigsäuremethylester oder Essigsäureethylester; eines Amids, wie N,N-Dimethylformamid, N,N-Dimethylacetamid, N-Methylformanilid, N-Methylpyrrolidon oder Hexamethylphosphorsäuretriamid; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether oder Diethylenglykolmonoethylether, und gegebenenfalls in Gegenwart eines basischen Katalysators, vorzugsweise eines Erdalkalimetall- oder Alkalimetallhydrides, -hydroxides, -amides, -alkoholates, -acetates, -carbonates oder -hydrogencarbonates, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amines, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), bei Temperaturen von -20°C bis 120°C, vorzugsweise bei Temperaturen von -10°C bis 80°C, umsetzt, und die so erhaltenen Enole der Formel (VIII), 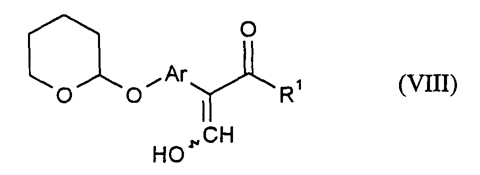 in welcher
- Rr und R¹: die oben angegebenen Bedeutungen haben,
vorzugsweise ohne weitere Aufarbeitung mit einer bereits weiter oben beschriebenen Halogenverbindung der Formel (III),
gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Ketons, wie Aceton, Butanon, Methyl-isobutylketon oder Cyclohexanon; eines Nitrils, wie Acetonitril, Propionitril, n- oder i-Butyronitril oder Benzonitril; eines Esters wie Essigsäuremethylester oder Essigsäureethylester; eines Amids, wie N,N-Dimethylformamid, N,N-Dimethylacetamid, N-Methylformanilid, N-Methylpyrrolidon oder Hexamethylphosphorsäuretriamid; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder reinem Wasser, und gegebenenfalls in Gegenwart einer Base, vorzugsweise eines Erdalkalimetall- oder Alkalimetallhydrides, -hydroxides, -amides, -alkoholates, -acetates, -carbonates oder -hydrogencarbonates, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amines, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), bei Temperaturen von-20°C bis 120°C, vorzugsweise bei Temperaturen von -10°C bis 80°C, umsetzt,oder wenn man (Verfahren d2) die oben bereits erwähnten Arylessigsäurederivate der Formel (VII), mit einem Alkalimetallnitrit, wie beispielsweise Natriumnitrit, oder vorzugsweise mit einem Alkylnitrit, wie beispielsweise t-Butylnitrit oder t-Amylnitrit, gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Ketons, wie Aceton, Butanon, Methyl-isobutylketon oder Cyclohexanon; eines Nitrils, wie Acetonitril, Propionitril, n- oder i-Butyronitril oder Benzonitril; eines Esters wie Essigsäuremethylester oder Essigsäureethylester; eines Amids, wie N,N-Dimethylformamid, N,N-Dimethylacetamid, N-Methylformanilid, N-Methylpyrrolidon oder Hexamethylphosphorsäuretriamid; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether oder Diethylenglykolmonoethylether, und gegebenenfalls in Gegenwart einer Base, vorzugsweise eines Erdalkalimetall- oder Alkalimetallhydrides, -hydroxides, -amides, -alkoholates, -acetates, -carbonates oder -hydrogencarbonates, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amines, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), bei Temperaturen von -50°C bis 100°C, bevorzugt von -20°C bis 50°C, umsetzt, und die so erhaltenen Oxime der Formel (IX), 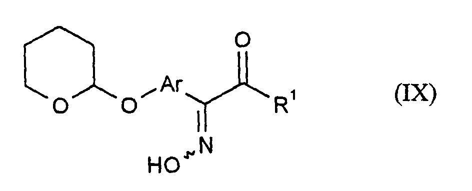 in welcher
- Ar und R¹: die oben angegebenen Bedeutungen haben,
vorzugsweise ohne weitere Aufarbeitung mit einer bereits weiter oben beschriebenen Halogenverbindung der Formel (III),
gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Ketons, wie Aceton, Butanon, Methylisobutylketon oder Cyclohexanon; eines Nitrils, wie Acetonitril, Propionitril, n- oder i-Butyronitril oder Benzonitril; eines Esters wie Essigsäuremethylester oder Essigsäureethylester; eines Amids, wie N,N-Dimethylformamid, N,N-Dimethylacetamid, N-Methylformanilid, N-Methylpyrrolidon oder Hexamethylphosphorsäuretriamid; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder reinem Wasser, und gegebenenfalls in Gegenwart einer Base, vorzugsweise eines Erdalkalimetall- oder Alkalimetallhydrides, -hydroxides, -amides, -alkoholates, -acetates, -carbonates oder -hydrogencarbonates, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amines, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), bei Temperaturen von -20°C bis 120°C, vorzugsweise bei Temperaturen von -10°C bis 80°C, umsetzt.

Die zur Durchführung der erfindungsgemäßen Verfahren d1) und d2) zur Herstellung der Tetrahydropyranylether der Formel (VI) als Ausgangsstoffe benötigten Arylessigsäurederivate sind durch die Formel (VII) allgemein definiert. In dieser Formel (VII) haben Ar und R¹ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Ar und R¹ angegeben wurden.

Die Arylessigsäurederivate der Formel (VII) sind bekannt und/oder können nach bekannten Verfahren hergestellt werden (vergleiche z. B. J. Org. Chem. 1994, 203-13).

Die zur Durchführung des erfindungsgemäßen Verfahrens b) weiterhin als Ausgangsstoffe benötigten Arylverbindungen sind durch die Formel (V) allgemein definiert. In dieser Formel (V) hat Ar¹ vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für Ar¹ angegeben wurde. V steht für Halogen, vorzugsweise für Fluor oder Chlor, oder für Z-T-, wobei Z und T vorzugsweise bzw. insbesondere diejenige Bedeutung haben, die bereits im Zusammenhang mit der Beschreibung der erfindungsgemäßen Verbindungen der Formel (I) als bevorzugt bzw. als insbesondere bevorzugt für Z und T angegeben wurde. X² steht für Halogen, vorzugsweise für Fluor oder Chlor, oder für Alkylsulfonyl oder Arylsulfonyl, vorzugsweise für Methylsulfonyl, Benzylsulfonyl oder Tolylsulfonyl.

Die Arylverbindungen der Formel (V) sind bekannte Synthesechemikalien und/oder können nach bekannten Verfahren hergestellt werden (vergleiche z. B. J. Org. Chem. 1994, 203-13, J. Heterocyclic Chem. 1993, 357 und Khim.-Farm. Zh. (1989), 23(6), 705-7).

Als Verdünnungsmittel zur Durchführung der erfindungsgemäßen Verfahren a) und b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die erfindungsgemäßen Verfahren a) und b) werden gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahrens a) und b) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens a) zur Herstellung der Verbindungen der Formel (I) setzt man pro Mol der Hydroxyverbindung der Formel (II) im allgemeinen 1 bis 15 Mol, vorzugsweise 1 bis 8 Mol an Halogenverbindung der Formel (III) ein.

Zur Durchführung des erfindungsgemäßen Verfahrens b) zur Herstellung der Verbindungen der Formel (I) setzt man pro Mol der Hydroxyarylverbindungen der allgemeinen Formel (IV) im allgemeinen 0,2 bis 5 Mol, vorzugsweise 0,5 bis 2 Mol an Arylverbindung der allgemeinen Formel (V) ein.

Die erfindungsgemäßen Verfahren a) und b) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vgl. auch die Herstellungsbeispiele).

Die erfindungsgemäßen Wirkstoffe weisen eine starke mikrobizide Wirkung auf und werden zur Bekämpfung von unerwünschten Mikroorganismen praktisch eingesetzt. Die Wirkstoffe sind für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei werden die erfindungsgemäßen Wirkstoffe mit besonders gutem Erfolg zur Bekämpfung von Getreidekrankheiten, wie beispielsweise gegen Erysiphe-Arten, Leptosphaeria-, sowie Pyrenophora-Arten, oder von Krankheiten im Wein-, Obst- und Gemüseanbau, wie beispielsweise gegen Podospherea-Arten, oder auch von Reiskrankheiten, wie beispielsweise Pyricularia-Arten, eingesetzt. Außerdem zeigen die erfindungsgemäßen Wirkstoffe eine besonders starke und breite in vitro Wirkung.

Die Wirkstoffe werden in Abhängigkeit von ihren jeweiligen physikalischen und/oder chemischen Eigenschaften gegebenenfalls in übliche Formulierungen übergeführt, wie z. B. Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es werden in den Formulierungen gegebenenfalls Haftmittel wie Carboxymethylcellulose, natürliche und synthetische, pulverige, körnige oder latexförmige Polymere verwendet, wie z. B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Gegebenenfalls werden Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink zugesetzt.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen.

In vielen Fällen werden dabei synergistische Wirkungen beobachtet.

Für die Mischungen kommen beispielsweise in Frage:

**Fungizide:**
2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb,
Difenoconazol, Dimethirimol, Dimethomorph, Diniconazol, Dinocap, Diphe-nylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropi-morph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil, Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

**Bakterizide:**
Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**
Abamectin, Abamectin, AC 303 630, Acephat, Acrinathrin, Alanycarb, Aldicarb, Alphamethrin, Amitraz, Avermectin, AZ 60541, Azadirachtin, Azinphos A,
Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin,
Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate,
Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos,
Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet,
Phosphamdon, Phoxim, Pirimicarb, Pirimiphos M, ,Primiphos A, Profenofos,
Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Gegebenenfalls werden die erfindungsgemäßen Wirkstoffe auch mit anderen bekannten Wirkstoffen, wie Herbiziden oder auch mit Düngemitteln und Wachstumsregulatoren gemischt.

Die Wirkstoffe werden als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verstäuben, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1:

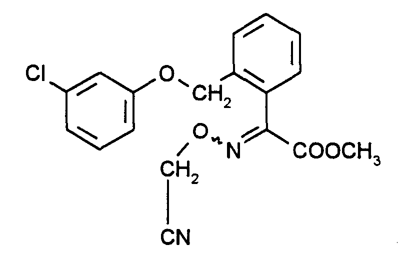

### Verfahren a)

Zu einer Lösung von 1,6 g (0,005 Mol) 2-Hydroximino-2-[2-(3-chlorphenoxymethyl)-phenyl]-acetamid in 50 ml Tetrahydrofuran gibt man unter Rühren nacheinander 0,67 g (0,006 Mol) Kalium-t-butylat und 0,35 g (0,0055 Mol) Chloracetonitril und rührt 2 Stunden bei 20°C. Die Mischung wird kurz bis zum Sieden erwärmt und ohne weitere Wärmezufuhr noch weitere 18 Stunden gerührt. Anschließend wird auf Wasser gegossen und mit Methyl-t-butylether extrahiert. Die organische Phase wird über Magnesiumsulfat getrocknet, filtriert und im Vakuum eingeengt. Der Rückstand wird mit Diisopropylether verrührt und abgesaugt. Man erhält 1,1 g (61,4 % der Theorie) an 2-Cyanmethoximino-2-[2-(2-methylphenoxymethyl)-phenyl]-acetamid.

¹H-NMR (CDCl₃, TMS): δ = 3,84 (s, 3H); 4,81 (s, 2H); 4,96 (s, 2H) ppm

### Beispiel 2:

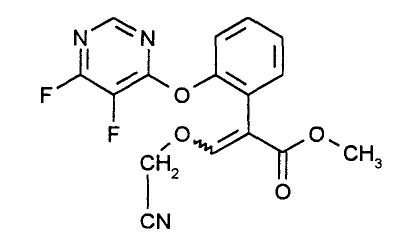

### Verfahren b)

Zu einer auf -10°C gekühlten Lösung von 3 g (13 mmol) 2-(2-Hydroxyphenyl)-3-cyanomethoxy-acrylsäuremethylester in 50 ml Dimethylformamid gibt man 1,74 g (13 mmol) 4,5,6-Trifluorpyrimidin und 0,43 g (14.3 mmol) 80%ige Natriumhydridsuspension. Die Lösung wird ohne weitere Kühlung gerührt, wobei sie sich innerhalb einer Stunde auf 20°C erwärmt. Man rührt weitere 2 Stunden bei 20°C, gießt die Lösung auf 100 ml Wasser und extrahiert zwei mal mit je 150 ml Methyl-t-butylether. Die vereinigten organischen Phasen werden über Natriumsulfat getrocknet und im Vakuum eingeengt. Man erhält 4,4 g (97,8 % der Theorie) 2-[2-(5,6-Trifluorpyrimid-4-yloxy)-phenyl]-3-cyanomethoxy-acrylsäuremethylester.

¹H-NMR (CDCl₃, TMS): δ = 3,67 (s, 3H), 4,63 (s, 2H).

### Beispiel 3:

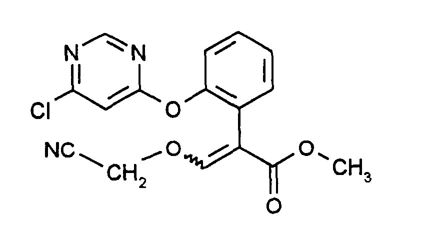

### Verfahren b)

Zu einer auf -10°C gekühlten Lösung von 3 g (13 mmol) 2-(2-Hydroxyphenyl)-3-cyanomethoxy-acrylsäuremethylester in 50 ml Dimethylformamid gibt man 1,94 g (13 mmol) 4,6-Dichlorpyrimidin und 0,43 g (14.3 mmol) 80%ige Natriumhydridsuspension. Die Lösung wird ohne weitere Kühlung gerührt, wobei sie sich innerhalb einer Stunde auf 20°C erwärmt. Man rührt weitere 2 Stunden bei 20°C, girßt die Lösung auf 100 ml Wasser und extrahiert zwei mal mit je 150 ml Methyl-t-butylether. Die vereinigten organischen Phasen werden über Natriumsulfat getrocknet und im Vakuum eingeengt. Man erhält 3,9 g (86,7 % der Theorie) 2-[2-(6-Chlorpyrimid-4-yloxy)-phenyl]-3-cyanomethoxy-acrylsäuremethylester.

¹H-NMR (CDCl₃, TMS): δ = 3,64 (s, 3H), 4,61 (s, 2H).

### Herstellung des Ausgangsproduktes,

### Beispiel (IV-1):

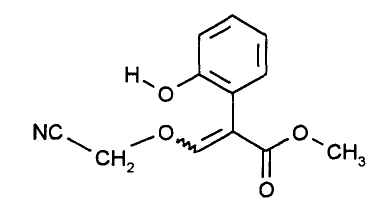

### Verfahren c)

Eine Lösung von 14,4 g (45,3 mmol) 2-[2-(Tetrahydropyran-2-yloxy)-phenyl]-3-cyanomethoxy-acrylsäuremethylester in 50 ml Methanol wird mit 5 g saurem Ionenaustauscher versetzt und 3 Stunden bei 20°C gerührt. Der Ionenaustauscher wird abfiltriert und das Filtrat im Vakuum eingeengt. Der Rückstand wird mit Dichlormethan/Essigsäureethylester (10:1) an Kieselgel chromatografiert. Man erhält 7,8 g (74% der Theorie) 2-(2-Hydroxyphenyl)-3-cyanomethoxy-acrylsäuremethylester.

¹H-NMR (CDCl₃, TMS): δ = 3,80 (s, 3H), 4,63 (s, 2H).

### Herstellung des Vorproduktes,

### Beispiel (VI-1):

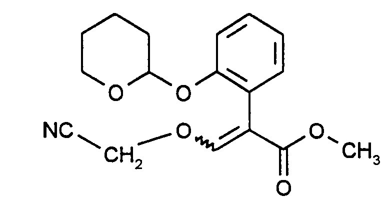

### Verfahren d1)

Zu einer Suspension von 1,96 g (66 mmol) 80%igem Natriumhydrid in 50 ml Dimethylformamid tropft man bei 20°C eine Lösung von 15 g (60 mmol) 2-[2-(Tetrahydropyran-2-yloxy)-phenyl]-acrylsäuremethylester in 36 ml Ameisensäuremethylester. Zu dieser Mischung gibt man nach 4 Stunden 15g Kaliumcarbonat und 9 g Chloracetonitril und rührt weitere 14 Stunden bei 20°C und gießt anschließend auf Wasser. Die Reaktionsmischung wird mit Essigsäureethylester extrahiert, die organische Phase über Natriumsulfat getrocknet und im Vakuum eingeengt. Man erhält 15,9g (83,55% der Theorie) 2-[2-(Tetrahydropyran-2-yloxy)-phenyl]-3-cyanomethoxy-acrylsäuremethylester.

¹H-NMR (CDCl₃, TMS): δ = 3,72 (s, 3H), 4,63 (s, 2H), 5,42 (m, 1H).

### Beispiel 4:

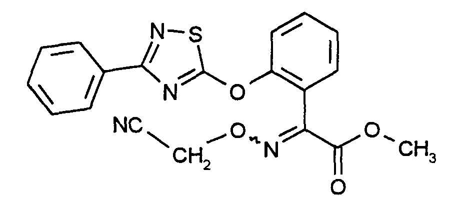

### Verfahren b)

Zu einer auf 0°C gekühlten Lösung von 1,4 g (6 mmol) 2-(2-Hydroxyphenyl)-2-cyanomethoximino-essigsäuremethylester und 1,9 g (6 mmol) 3-Phenyl-5-(4-tolylsulfonyl)-1,2,4-thiadiazol in 20 ml Dimethylformamid gibt man 0,2 g (6 mmol) 80%ige Natriumhydridsuspension und rührt 48 Stunden lang ohne weitere Kühlung. Die Reaktionsmischung wird in Essigsäureethylester aufgenommen, mit Wasser gewaschen, über Natriumsulfat getrocknet und im Vakuum eingeengt. Man erhält 0,8 g (34 % der Theorie) 2-[2-(3-Phenyl-1,2,4-thiadiazol-5-yloxy)-phenyl]-2-cyanomethoximino-essigsäuremethylester.

¹H-NMR (CDCl₃, TMS): δ = 3,80 (s, 3H), 4,80(s, 2H).

### Herstellung des Ausgangsproduktes,

### Beispiel (IV-2):

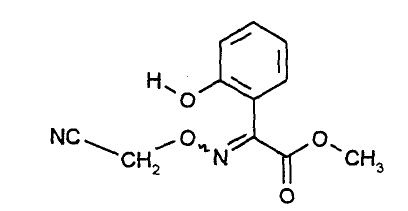

### Verfahren c)

Eine Lösung von 3,2 g (10 mmol) 2-[2-(Tetrahydropyran-2-yloxy)-phenyl]-2-cyanomethoximino-essigsäuremethylester in 20 ml Methanol wird mit 0,1 g saurem Ionenaustauscher versetzt und 18 Stunden bei 20°C gerührt. Der Ionenaustauscher wird abfiltriert und das Filtrat im Vakuum eingeengt. Der Rückstand wird mit Hexan/Aceton (7:3) an Kieselgel chromatografiert. Man erhält 1,4 g (60% der Theorie) 2-(2-Hydroxyphenyl)-2-cyanomethoximino-essigsäuremethylester.

¹H-NMR (CDCl₃, TMS): δ = 3,95 (s, 3H), 4,90 (s, 2H).

### Herstellung des Vorproduktes,

### Beispiel (VI-2):

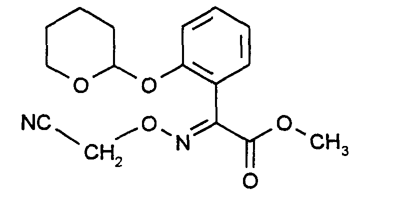

### Verfahren d2)

Zu einer Lösung von 12,3 g (110 mmol) Kalium-t-butylat in 120 ml t-Butanol tropft man eine Lösung von 34,3 g (300 mmol) t-Butylnitrit und 25 g (100 mmol) 2-[2-(Tetrahydropyran-2-yloxy)-phenyl]-acrylsäuremethylester in 30 ml t-Butanol, wobei sich die Reaktionslösung auf 45°C erwärmt. Zu dieser Mischung gibt man nach 2 Stunden 13,2 g (110 mmol) Bromacetonitril und rührt weitere 18 Stunden bei 20°C. Die Reaktionsmischung wird im Vakuum eingeengt, mit Methyl-t-butylether aufgenommen, mit Wasser gewaschen, über Natriumsulfat getrocknet und im Vakuum eingeengt. Der Rückstand wird mit Hexan/Aceton (8:2) an Kieselgel chromatografiert. Man erhält 14,9 g (46,9% der Theorie) 2-[2-(Tetrahydropyran-2-yloxy)-phenyl]-3-cyanomethoxy-acrylsäuremethylester.

¹H-NMR (CDCl₃, TMS): δ = 3,9 (s, 3H), 4,85 (s, 2H).
Analog den Beispielen (1-4), sowie entsprechend der allgemeinen Beschreibung des erfindungsgemäßen Herstellungsverfahrens, erhält man auch die in der nachstehenden Tabelle 17 aufgeführten erfindungsgemäßen Verbindungen der Formel (I):

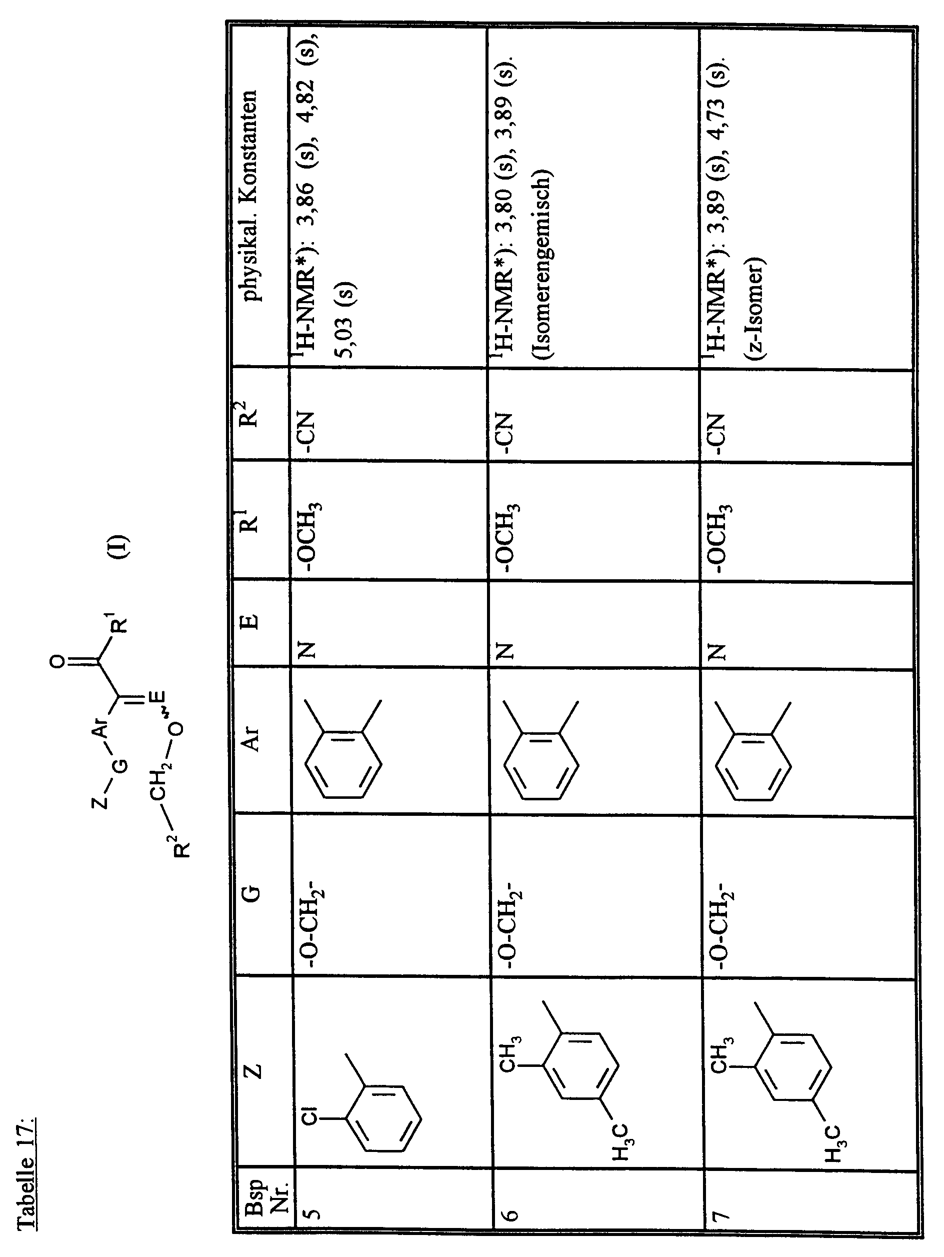

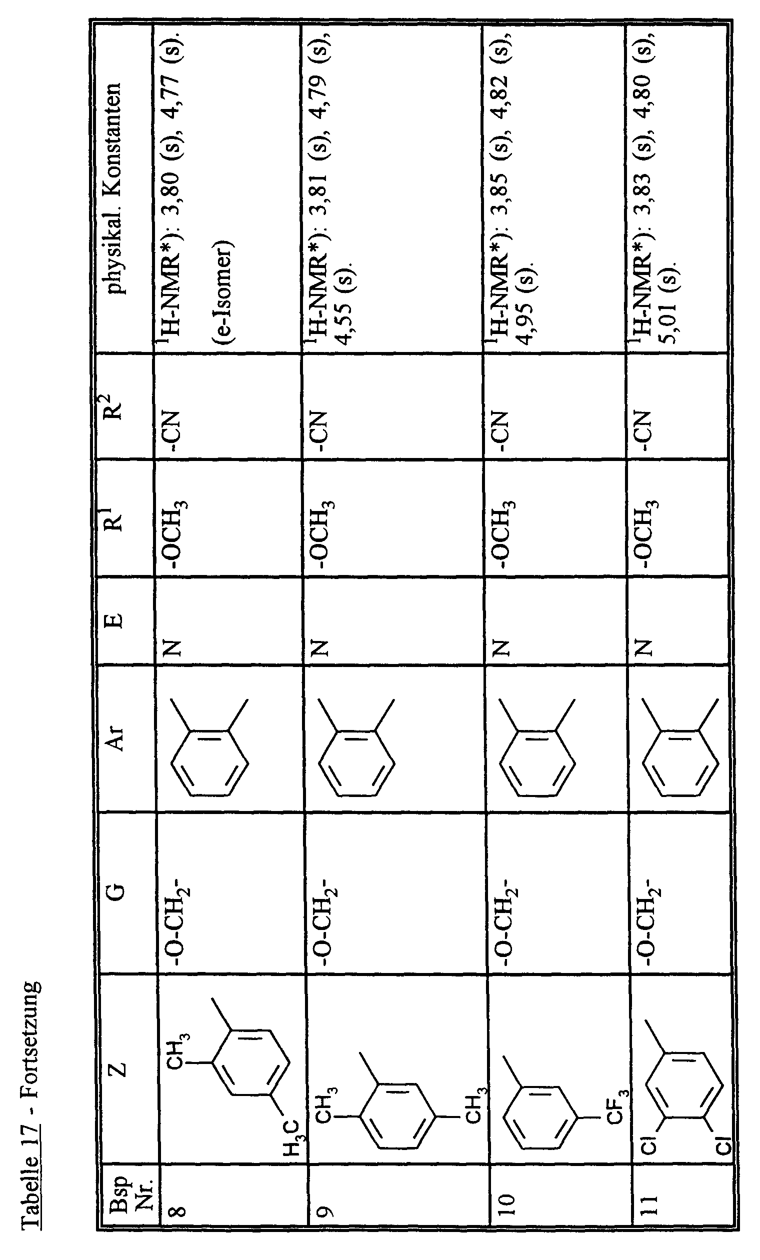

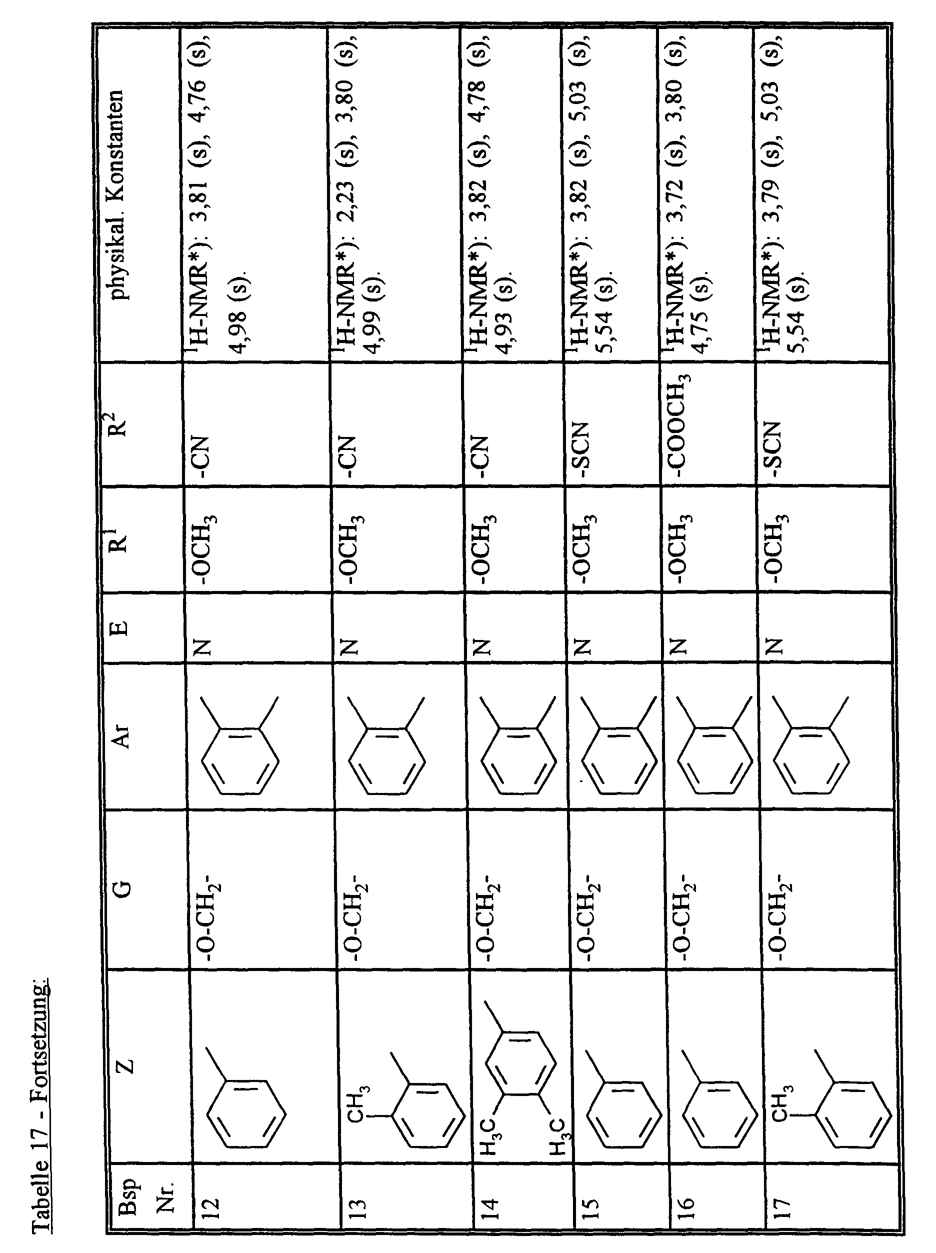

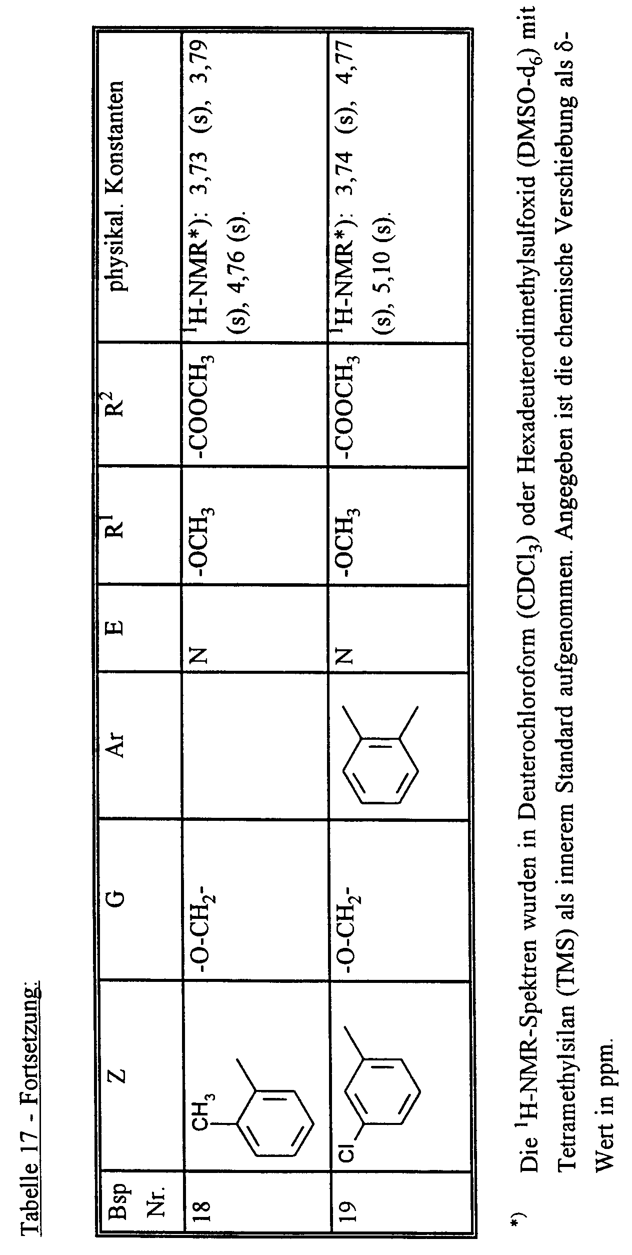

### Anwendungsbeispiele:

In den folgenden Anwendungsbeispielen wird die nachstehend aufgeführte Verbindung als Vergleichssubstanz eingesetzt: 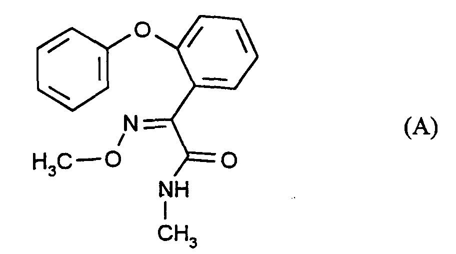 (E)-.alpha.-(methoxyimino)-N-methyl-2-phenoxy-benzeneacetamide

### Beispiel A

### Podosphaera-Test (Apfel) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung.

Bei diesem Test zeigen z.B. die folgenden Verbindungen der Herstellungsbeispiele (6), (7), (8) und (9) bei einem Wirkstoffkonzentration von 100 ppm einen Wirkungsgrad von 82 bis 99 %.

### Beispiel B

### Leptosphaeria nodorum -Test (Weizen) / kurativ

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Sporensuspension von Leptosphaeria nodorum besprüht. Die Pflanzen verbleiben 48 Stunden bei 20°C und 100% relativer Luftfeuchtigkeit in einer Inkubationskabine. Anschließend besprüht man die Pflanzen mit der Wirkstoffzubereitung in der angegebenen Aufwandmenge.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Bei diesem Test zeigen z.B. die folgenden Verbindungen der Herstellungsbeispiele (7), (8) und (9) bei einem Wirkstoffaufwandmenge von 125 g/ha einen Wirkungsgrad von 50 %.

## Patentansprüche

1. Verbindungen der Formel (I), 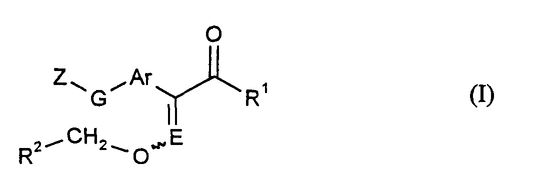 in welcher
Ar für jeweils gegebenenfalls substituiertes Arylen oder Heteroarylen steht,
E für =CH- oder Stickstoff steht,
G für eine Einfachbindung, für Sauerstoff, Schwefel oder für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen -Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- oder -T-Ar¹-Q- steht, wobei
Ar¹ für gegebenenfalls substituiertes Arylen, Heteroarylen, Cycloalkylen oder Heterocycloalkylen steht,
n für die Zahlen 0, 1 oder 2 steht,
Q für Sauerstoff oder Schwefel steht,
R³ fürWasserstoff, Cyano oder jeweils gegebenenfalls substituiertes Alkyl, Alkoxy, Alkylthio, Alkylamino, Dialkylamino oder Cycloalkyl steht, und
R⁴ für Wasserstoff, Hydroxy, Cyano oder jeweils gegebenenfalls substituiertes Alkyl, Alkoxy oder Cycloalkyl steht, und
R⁵ für Wasserstoff oder Alkyl steht, und
T für eine Einfachbindung, für Sauerstoff, Schwefel, -CH₂-O-, -CH₂-S- oder für gegebenenfalls substituiertes Alkandiyl steht,
R¹ für Alkoxy, Alkylamino oder Dialkylamino steht,
R² für Cyano, Thiocyanato, Alkylcarbonyl, Alkoxycarbonyl, Aminocarbonyl, Aminothiocarbonyl, Alkylaminocarbonyl oder Dialkylaminocarbonyl steht, und
Z für jeweils gegebenenfalls substituiertes Alkyl, Alkenyl, Alkinyl, Cycloalkyl, Aryl oder Heterocyclyl steht.

2. Verbindungen der Formel (I) gemäß Anspruch 1, in welcher
Ar für jeweils gegebenenfalls substituiertes Phenylen oder Naphthylen, für mono- oder bicyclisches Heteroarylen mit jeweils 5 oder 6 Ringgliedern oder für benzokondensiertes Heteroarylen mit 5 oder 6 Ringgliedern steht, von denen jeweils mindestens eines für Sauerstoff, Schwefel oder Stickstoff steht und gegebenenfalls ein oder zwei weitere für Stickstoff stehen, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
E steht für =CH- oder Stickstoff,
G steht für eine Einfachbindung, für Sauerstoff, Schwefel oder für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen
-Q-CQ-, -CQ-Q-, -CH₂-Q-; -Q-CH₂-, -CQ-Q-CH₂-, -CH₂-Q-CQ--Q-CQ-CH₂-, -Q-CQ-Q-CH₂-, -N=N-, -S(O)ₙ-, -CH₂-S(O)ₙ-, -CQ-, -(O)ₙ-CH₂-, -C(R³)=N-O-, -C(R³)=N-CH₂-, -N(R⁴), -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- oder -T-Ar¹-Q- steht, wobei
n für die Zahlen 0, 1 oder 2 steht,
Q für Sauerstoff oder Schwefel steht,
R³ für Wasserstoff, Cyano, für jeweils gegebenenfalls durch Halogen, Cyano oder C₁-C₁-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₁-Alkyl oder C₁-C₁-Alkoxy-carbonyl substituiertes Cycloalkyl mit 3 bis 6 Kohlenstoffatomen steht, und
R⁴ für Wasserstoff, Hydroxy, Cyano oder für gegebenenfalls durch Halogen, Cyano oder C₁-C₁-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R⁵ für Wasserstoff oder Alkyl mit 1 bis 4 Kohlenstoffatomen steht,
Ar¹ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie;
Cycloalkyl mit 3 bis 6 Kohlenstoffatomen und
T für eine Einfachbindung, für Sauerstoff, Schwefel, -CH₂-O-, -CH₂-S- oder für Alkandiyl mit 1 bis 3 Kohlenstoffatomen steht,
R¹ steht für Alkyl, Alkoxy, Alkylamino oder Dialkylamino mit 1 bis 4 Kohlenstoffatomen in den jeweiligen Alkylketten,
R² steht für Cyano, Thiocyanato, Aminocarbonyl, Aminothiocarbonyl, oder für Alkylcarbonyl, Alkoxycarbonyl, Alkylaminocarbonyl oder Dialkylaminocarbonyl mit 1 bis 4 Kohlenstoffatomen in den jeweiligen Alkylketten,
Z steht für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen;
für jeweils gegebenenfalls durch Halogen substituiertes Alkenyl oder Alkinyl mit jeweils bis zu 8 Kohlenstoffatomen;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen;
für jeweils gegebenenfalls einfach oder mehrfach, gleich oder verschieden substituiertes Phenyl, Naphthyl oder für Heterocyclyl mit 3 bis 7 Ringgliedern, von denen mindestens eines für Sauerstoff, Schwefel oder Stickstoff und gegebenenfalls ein oder zwei weitere für Stickstoff stehen, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -
oder eine Gruppierung 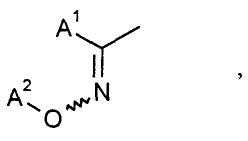 worin
A¹ für Alkyl mit 1 bis 4 Kohlenstoffatomen oder Cycloalkyl mit 1 bis 6 Kohlenstoffatomen steht und
A² für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht.

3. Verbindungen der Formel (I) gemäß Anspruch 1, in welcher
Ar für jeweils gegebenenfalls einfach oder zweifach durch Fluor, Chlor, Cyano, Methyl, Ethyl, Cyclopropyl, Trifluormethyl, Methoxy, Ethoxy, Methylthio, Methylsulfinyl oder Methylsulfonyl substituiertes ortho-, meta- oder para-Phenylen, Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht,
E steht insbesondere für =CH- oder Stickstoff,
G steht für Sauerstoff oder für jeweils gegebenenfalls durch Fluor, Chlor oder Brom substituiertes Dimethylen (Ethan-1,2-diyl), Ethen-1,2-diyl oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- oder -T-Ar¹-Q- steht, wobei
n für die Zahlen 0, 1 oder 2 steht,
Q für Sauerstoff oder Schwefel steht,
R³ für Wasserstoff, Cyano, Methyl, Ethyl oder Cyclopropyl steht und
R⁴ für Wasserstoff, Methyl, Ethyl oder Cyclopropyl steht,
R⁵ für Wasserstoff oder Alkyl mitl bis 4 Kohlenstoffatomen steht,
Ar¹ für jeweils gegebenenfalls einfach bis dreifach, gleich oder verschieden substituiertes Phenylen oder Pyridindiyl, für jeweils gegebenenfalls einfach substituiertes Pyrimidindiyl, Pyridazindiyl, Pyrazindiyl, 1,2,3-Triazindiyl, 1,2,4-Triazindiyl oder 1,3,5-Triazindiyl oder für 1,2,4-Thiadiazoldiyl, 1,3,4-Thiadiazoldiyl, 1,2,4-Oxadiazoldiyl, 1,3,4-Oxadiazoldiyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
T für eine Einfachbindung, für Sauerstoff, Schwefel, -CH₂-O-, CH₂-S-, Methylen, Ethylen oder Propylen steht.
R¹ steht für Methyl, Ethyl, n- oder i-Propyl, Methoxy, Ethoxy, n- oder i-Propoxy-, Methylamino, Ethylamino, n- oder i-Propylamino, Dimethylamino, Diethylamino oder N-Methyl-N-ethylamino,
R² steht für Cyano, Thiocyanato, Aminocarbonyl, Aminothiocarbonyl, Acetyl, Propionyl, Pivaloyl, Methoxycarbonyl, Ethoxycarbonyl, Methylaminocarbonyl, Ethylaminocarbonyl oder Dimethylaminocarbonyl,
Z steht für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
oder eine Gruppierung 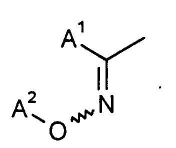
A¹ steht für Methyl, Ethyl, n- oder i-Propyl, n-, i-, s- oder t-Butyl, Cyclopropyl oder Cyclobutyl,
A² steht für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl.

4. Verbindungen der Formel (I) gemäß Anspruch 1,
in welcher
Ar für ortho-Phenylen, Pyridin-2,3-diyl oder Thiophen-2,3-diyl steht,
E für =CH- oder Stickstoff steht,
G für -O-CH₂- steht,
R¹ für Methoxy oder Methylamino steht,
R² für Cyano, Thiocyanato, Aminocarbonyl, Aminothiocarbonyl, Acetyl, Propionyl, Pivaloyl, Methoxycarbonyl, Ethoxycarbonyl, Methylaminocarbonyl, Ethylaminocarbonyl oder Dimethylaminocarbonyl steht, und
Z für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy
oder eine Gruppierung 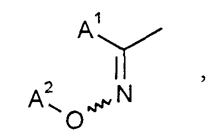 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

5. Verbindungen der Formel (I) gemäß Anspruch 1,
in welcher
Ar für ortho-Phenylen, Pyridin-2,3-diyl oder Thiophen-2,3-diyl steht,
E für =CH- oder Stickstoff steht,
G für -C(R³)=N-O-CH₂- steht,
R³ für Methyl oder Cyclopropyl steht,
R¹ für Methoxy oder Methylamino steht,
R² für Cyano, Thiocyanato, Aminocarbonyl, Aminothiocarbonyl, Acetyl, Propionyl, Pivaloyl, Methoxycarbonyl, Ethoxycarbonyl, Methylaminocarbonyl, Ethylaminocarbonyl oder Dimethylaminocarbonyl steht, und
Z für jeweils gegebenenfalls einfach bis dreifach, gleich oder verschieden substituiertes Phenyl, Pyridyl oder Pyrimidyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy.

6. Verbindungen der Formel (I) gemäß Anspruch 1,
in welcher
Ar für ortho-Phenylen steht,
E für =CH- oder Stickstoff steht,
G für Sauerstoff oder -T-Ar¹-O- steht,
Ar¹ für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
R¹ für Methoxy oder Methylamino steht,
R² für Cyano, Thiocyanato, Aminocarbonyl, Aminothiocarbonyl, Acetyl, Propionyl, Pivaloyl, Methoxycarbonyl, Ethoxycarbonyl, Methylaminocarbonyl, Ethylaminocarbonyl oder Dimethylaminocarbonyl steht,
T für eine Einfachbindung, für Sauerstoff, Schwefel, -CH₂-O-, CH₂-S-, Methylen, Ethylen oder Propylen steht und
Z für jeweils gegebenenfalls einfach bis dreifach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy, Difluorchlormethoxy, Trifluorethoxy, Trifluormethoxy oder jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy, substituiertes Phenyl, Pyridyl, Pyrimidyl oder Thienyl steht.

7. Schädlingsbekämpfungsmittel, gekennzeichnet durch einen Gehalt an mindestens einer Verbindung der Formel (I) nach Anspruch 1.

8. Verfahren zur Bekämpfung von Schädlingen, dadurch gekennzeichnet, daß man Verbindungen der Formel (I) nach Anspruch 1 auf Schädlinge und/oder ihren Lebensraum einwirken läßt.

9. Verfahren zur Herstellung von Verbindungen der Formel (I) nach Anspruch 1, dadurch gekennzeichnet, daß man
a) Hydroxyverbindungen der allgemeinen Formel (II) 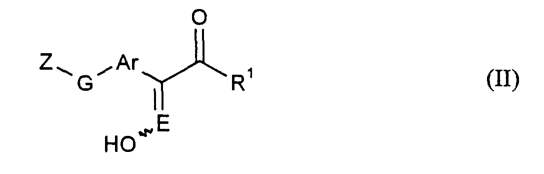 in welcher
Ar, E, G, R¹ und Z die in Anspruch 1 angegebenen Bedeutungen haben,
mit einer Halogenverbindung der allgemeinen Formel (III)
X¹-CH₂-R² (III)
in welcher
X¹ für Halogen steht und
R² die oben angegebene Bedeutung hat,
gegebenenfalls in Gegenwart eines Säureakzeptors und gegebenenfalls in Gegenwart eines Verdünnungsmittels umsetzt; oder wenn man
b) Hydroxyarylverbindungen der allgemeinen Formel (IV) 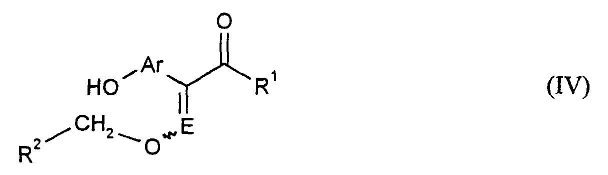 in welcher
Ar, E, R¹ und R² die in Anspruch 1 angegebenen Bedeutungen haben,
mit einer Arylverbindung der allgemeinen Formel (V) 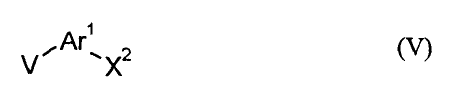 in welcher
Ar¹ die in Anspruch 1 angegebene Bedeutung hat,
V für Halogen oder Z-T- steht, wobei
Z und T die in Anspruch 1 angegebenen Bedeutungen haben und
X² für Halogen, Alkylsulfonyl oder Arylsulfonyl steht,
gegebenenfalls in Gegenwart eines Säureakzeptors und gegebenenfalls in Gegenwart eines Verdünnungsmittels umsetzt.

10. Verwendung von Verbindungen der Formel (I) nach den Ansprüchen 1 bis 6 zur Bekämpfung von Schädlingen.

11. Verfahren zur Herstellung von Schädlingsbekämpfungsmitteln, dadurch gekennzeichnet, daß man Verbindungen der Formel (I) nach den Ansprüchen 1 bis 6 mit Streckmitteln und/oder oberflächenaktiven Mitteln vermischt.

12. Verbindungen der Formel (IV) 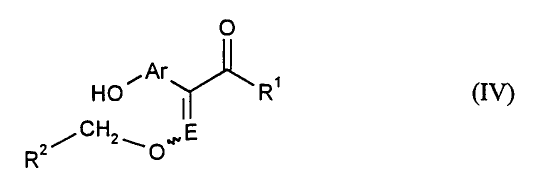 in welcher
Ar, E, R¹ und R² die in Anspruch 1 angegebenen Bedeutungen haben.
Verbindungen der Formel (IV) 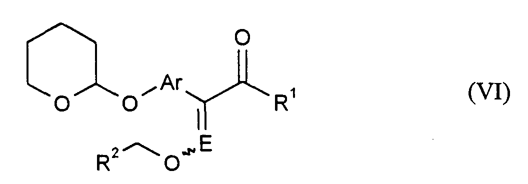 in welcher
Ar, E, R¹ und R² die in Anspruch 1 angegebenen Bedeutungen haben.

## Claims

1. Compounds of the formula (I) 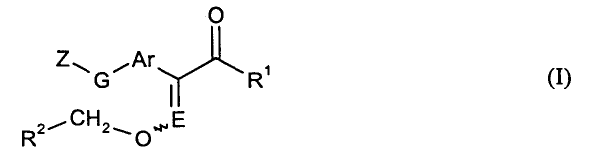 in which
Ar represents respectively optionally substituted arylene or heteroarylene,
E represents =CH- or nitrogen,
G represents a single bond, represents oxygen, sulphur or represents respectively optionally halogen-, hydroxyl-, alkyl-, halogenoalkyl- or cycloalkyl-substituted alkanediyl, alkenediyl, alkinediyl or one of the groupings below -Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- or -T-Ar¹-Q-, where
Ar¹ represents optionally substituted arylene, heteroarylene, cycloalkylene or heterocycloalkylene,
n represents the numbers 0, 1 or 2,
Q represents oxygen or sulphur,
R³ represents hydrogen, cyano or respectively optionally substituted alkyl, alkoxy, alkylthio, alkylamino, dialkylamino or cycloalkyl, and
R⁴ represents hydrogen, hydroxyl, cyano or respectively optionally substituted alkyl, alkoxy or cycloalkyl, and
R⁵ represents hydrogen or alkyl, and
T represents a single bond, represents oxygen, sulphur, -CH₂-O-, -CH₂-S- or represents optionally substituted alkanediyl,
R¹ represents alkoxy, alkylamino or dialkylamino,
R² represents cyano, thiocyanato, alkylcarbonyl, alkoxycarbonyl, aminocarbonyl, aminothiocarbonyl, alkylaminocarbonyl or dialkylaminocarbonyl, and
Z represents respectively optionally substituted alkyl, alkenyl, alkynyl, cycloalkyl, aryl or heterocyclyl.

2. Compounds of the formula (I) according to Claim 1 in which
Ar represents respectively optionally substituted phenylene or naphthylene, represents mono- or bicyclic heteroarylene having in each case 5 or 6 ring members or represents benzo-fused heteroarylene having 5 or 6 ring members, at least one of which represents oxygen, sulphur or nitrogen and one or two more optionally represent nitrogen, the possible substituents being selected from the list below:
halogen, cyano, nitro, amino, hydroxyl, formyl, carboxy, carbamoyl, thiocarbamoyl, respectively straight-chain or branched alkyl, alkoxy, alkylthio, alkylsulphinyl or alkylsulphonyl having in each case 1 to 6 carbon atoms, respectively straight-chain or branched alkenyl, alkenyloxy or alkinyloxy having in each case 2 to 6 carbon atoms, respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms, respectively straight-chain or branched halogenoalkenyl or halogenoalkenyloxy having in each case 2 to 6 carbon atoms and 1 to 11 identical or different halogen atoms, respectively straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties, alkylene or dioxyalkylene, each of which has 1 to 6 carbon atoms, is doubly attached and is optionally mono- or polysubstituted by identical or different halogens and/or straight-chain or branched alkyl having 1 to 4 carbon atoms and/or straight-chain or branched halogenoalkyl having 1 to 4 carbon atoms and 1 to 9 identical or different halogen atoms,
E represents =CH- or nitrogen,
G represents a single bond, represents oxygen, sulphur or represents respectively optionally halogen-, hydroxyl-, C₁-C₄-alkyl-, C₁-C₄-halogenoalkyl- or C₃-C₆-cycloalkyl-substituted alkanediyl, alkenediyl, alkinediyl having in each case up to 4 carbon atoms or one of the groupings below
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -(O)ₙ-CH₂-, -C(R³)=N-O-, -C(R³)=N-O-CH₂-, -N(R⁴), -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- or -T-Ar¹-Q-, where
n represents the numbers 0, 1 or 2,
Q represents oxygen or sulphur,
R³ represents hydrogen, cyano, represents respectively optionally halogen-, cyano- or C₁-C₄-alkoxy-substituted alkyl, alkoxy, alkylthio, alkylamino or dialkylamino having in each case 1 to 6 carbon atoms in the alkyl groups or represents respectively optionally halogen-, cyano-, carboxy-, C₁-C₄-alkyl- or C₁-C₄-alkoxy-carbonyl-substituted cycloalkyl having 3 to 6 carbon atoms, and
R⁴ represents hydrogen, hydroxyl, cyano or represents optionally halogen-, cyano- or C₁-C4-alkoxy-substituted alkyl having 1 to 6 carbon atoms or represents optionally halogen-, cyano-, carboxy-, C₁-C₄-alkyl- or C₁-C₄-alkoxy-carbonyl-substituted cycloalkyl having 3 to 6 carbon atoms, and
R⁵ represents hydrogen or alkyl having 1 to 4 carbon atoms,
Ar¹ represents phenylene, naphthylene, cycloalkylene, each of which is optionally mono- or polysubstituted by identical or different substituents, or represents heteroarylene or heterocycloalkylene having 3 to 7 ring members, at least one of which represents oxygen, sulphur or nitrogen and one or two more optionally represent nitrogen, the possible substituents being selected from the list below:
halogen, cyano, nitro, amino, hydroxyl, formyl, carboxy, carbamoyl, thiocarbamoyl;
respectively straight-chain or branched alkyl, alkoxy, alkylthio, alkylsulphinyl or alkylsulphonyl having in each case 1 to 6 carbon atoms;
respectively straight-chain or branched alkenyl or alkenyloxy having in each case 2 to 6 carbon atoms;
respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
respectively straight-chain or branched halogenoalkenyl or halogenoalkenyloxy having in each case 2 to 6 carbon atoms and 1 to 11 identical or different halogen atoms;
respectively straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties, and;
cycloalkyl having 3 to 6 carbon atoms and
T represents a single bond, represents oxygen, sulphur, -CH₂-O-, -CH₂-S- or represents alkanediyl having 1 to 3 carbon atoms,
R¹ represents alkyl, alkoxy, alkylamino or dialkylamino having 1 to 4 carbon atoms in the respective alkyl chains,
R² represents cyano, thiocyanato, aminocarbonyl, aminothiocarbonyl, or represents alkylcarbonyl, alkoxycarbonyl, alkylaminocarbonyl or dialkylaminocarbonyl having 1 to 4 carbon atoms in the respective alkyl chains,
Z represents alkyl having 1 to 8 carbon atoms which is optionally mono- or polysubstituted by identical or different substituents selected from the group consisting of halogen, cyano, hydroxyl, amino, C₁-C₄-alkoxy, C₁-C₄-alkylthio, C₁-C₄-alkylsulphinyl and C₁-C₄-alkylsulphonyl (each of which is optionally substituted by halogen);
represents respectively optionally halogen-substituted alkenyl or alkynyl having in each case up to 8 carbon atoms;
represents cycloalkyl having 3 to 6 carbon atoms which is optionally mono- or polysubstituted by identical or different substituents selected from the group consisting of halogen, cyano, carboxy, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl and C₁-C₄-alkoxycarbonyl;
represents phenyl, naphthyl, each of which is optionally mono- or polysubstituted by identical or different substituents, or represents heterocyclyl having 3 to 7 ring members, at least one of which represents oxygen, sulphur or nitrogen and one or two more optionally represent nitrogen, the possible substituents being selected from the list below:
halogen, cyano, nitro, amino, hydroxyl, formyl, carboxy, carbamoyl, thiocarbamoyl;
respectively straight-chain or branched alkyl, alkoxy, alkylthio, alkylsulphinyl or alkylsulphonyl having in each case 1 to 6 carbon atoms;
respectively straight-chain or branched alkenyl or alkenyloxy having in each case 2 to 6 carbon atoms;
respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
respectively straight-chain or branched halogenoalkenyl or halogenoalkenyloxy having in each case 2 to 6 carbon atoms and 1 to 11 identical or different halogen atoms;
respectively straight-chain or branched alkylamino, dialkylamino, alkylcarbonyl, alkylcarbonyloxy, alkoxycarbonyl or alkylsulphonyloxy, having in each case 1 to 6 carbon atoms in the individual alkyl moieties;
alkylene or dioxyalkylene, each of which has 1 to 6 carbon atoms, is doubly attached and is optionally mono- or polysubstituted by identical or different halogens and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms;
heterocyclyl or heterocyclyl-methyl having in each case 3 to 7 ring members, 1 to 3 of which are in each case identical or different hetero atoms - in particular nitrogen, oxygen and/or sulphur -
or a grouping 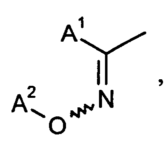 in which
A¹ represents alkyl having 1 to 4 carbon atoms or cycloalkyl having 1 to 6 carbon atoms and
A² represents optionally cyano-, alkoxy-, alkylthio-, alkylamino-, dialkylamino- or phenyl-substituted alkyl having 1 to 4 carbon atoms, alkenyl or alkynyl having in each case 2 to 4 carbon atoms.

3. Compounds of the formula (I) according to Claim 1 in which
Ar represents ortho-, meta- or para-phenylene, furandiyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl (in particular pyridine-2,3-diyl), pyrimidinediyl, pyridazinediyl, pyrazinediyl, 1,3,4-triazinediyl or 1,2,3-triazinediyl, each of which is optionally mono- or disubstituted by fluorine, chlorine, cyano, methyl, ethyl, cyclopropyl, trifluoromethyl, methoxy, ethoxy, methylthio, methylsulphinyl or methylsulphonyl,
E represents =CH- or nitrogen,
G represents oxygen or represents respectively optionally fluorine-, chlorine- or bromine-substituted dimethylene (ethane-1,2-diyl), ethene-1,2-diyl or one of the groupings below
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- or -T-Ar¹-Q-, where
n represents the numbers 0, 1 or 2,
Q represents oxygen or sulphur,
R³ represents hydrogen, cyano, methyl, ethyl or cyclopropyl and
R⁴ represents hydrogen, methyl, ethyl or cyclopropyl,
R⁵ represents hydrogen or alkyl having 1 to 4 carbon atoms,
Ar¹ represents phenylene or pyridinediyl, each of which is optionally mono- to trisubstituted by identical or different substituents, represents respectively optionally monosubstituted pyrimidinediyl, pyridazinediyl, pyrazinediyl, 1,2,3-triazinediyl, 1,2,4-triazinediyl or 1,3,5-triazinediyl or represents 1,2,4-thiadiazolediyl, 1,3,4-thiadiazolediyl, 1,2,4-oxadiazolediyl, 1,3,4-oxadiazolediyl, the possible substituents preferably being selected from the list below:
fluorine, chlorine, bromine, cyano, methyl, ethyl, n- or i-propyl, cyclopropyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl and
T represents a single bond, represents oxygen, sulphur, -CH₂-O-, CH₂-S-, methylene, ethylene or propylene.
R¹ represents methyl, ethyl, n- or i-propyl, methoxy, ethoxy, n- or i-propoxy, methylamino, ethylamino, n- or i-propylamino, dimethylamino, diethylamino or N-methyl-N-ethylamino,
R² represents cyano, thiocyanato, aminocarbonyl, aminothiocarbonyl, acetyl, propionyl, pivaloyl, methoxycarbonyl, ethoxycarbonyl, methylaminocarbonyl, ethylaminocarbonyl or dimethylaminocarbonyl,
Z represents phenyl, 1,2,4-thiadiazolyl, 1,3,4-thiadiazolyl, 1,2,4-oxadiazolyl, 1,3,4-oxadiazolyl, pyridinyl, pyrimidyl, pyridazinyl, pyrazinyl, 1,2,3-triazinyl, 1,2,4-triazinyl or 1,3,5-triazinyl, each of which is optionally mono- to trisubstituted by identical or different substituents, the possible substituents being selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl,
methylenedioxy, ethylenedioxy, each of which is doubly attached and each of which is optionally mono- to tetrasubstituted by identical or different substituents selected from the group consisting of fluorine, chlorine, methyl, trifluoromethyl and ethyl
or a grouping 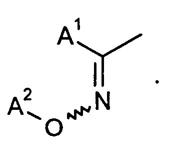
A¹ represents methyl, ethyl, n- or i-propyl, n-, i-, s- or t-butyl, cyclopropyl or cyclobutyl,
A² represents methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl.

4. Compounds of the formula (I) according to Claim 1
in which
Ar represents ortho-phenylene, pyridine-2,3-diyl or thiophene-2,3-diyl,
E represents =CH- or nitrogen,
G represents -O-CH₂-,
R¹ represents methoxy or methylamino,
R² represents cyano, thiocyanato, aminocarbonyl, aminothiocarbonyl, acetyl, propionyl, pivaloyl, methoxycarbonyl, ethoxycarbonyl, methylaminocarbonyl, ethylaminocarbonyl or dimethylaminocarbonyl, and
Z represents phenyl which is optionally mono- to trisubstituted by identical or different substituents, the possible substituents preferably being selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl,
methylenedioxy or ethylenedioxy, each of which is doubly attached and each of which is optionally mono- to tetrasubstituted by identical or different substituents selected from the group consisting of fluorine, chlorine, methyl, trifluoromethyl or ethyl
or a grouping 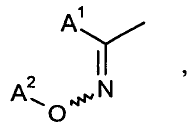 in which
A¹ represents methyl, ethyl, n- or i-propyl, n-, i-, s- or t-butyl, cyclopropyl or cyclobutyl and
A² represents methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl.

5. Compounds of the formula (I) according to Claim 1
in which
Ar represents ortho-phenylene, pyridine-2,3-diyl or thiophene-2,3-diyl,
E represents =CH- or nitrogen,
G represents -C(R³)=N-O-CH₂-,
R³ represents methyl or cyclopropyl,
R¹ represents methoxy or methylamino,
R² represents cyano, thiocyanato, aminocarbonyl, aminothiocarbonyl, acetyl, propionyl, pivaloyl, methoxycarbonyl, ethoxycarbonyl, methylaminocarbonyl, ethylaminocarbonyl or dimethylaminocarbonyl, and
Z represents phenyl, pyridyl or pyrimidyl, each of which is optionally mono- to trisubstituted by identical or different substituents, the possible substituents preferably being selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl,
methylenedioxy or ethylenedioxy, each of which is doubly attached and each of which is optionally mono- to tetrasubstituted by identical or different substituents selected from the group consisting of fluorine, chlorine, methyl, trifluoromethyl and ethyl.

6. Compounds of the formula (I) according to Claim 1
in which
Ar represents ortho-phenylene,
E represents =CH- or nitrogen,
G represents oxygen or -T-Ar¹-O-,
Ar¹ represents 1,2,4-thiadiazolediyl, 1,3,4-thiadiazolediyl, 1,2,4-oxadiazolediyl, 1,3,4-oxadiazolediyl or represents pyridinediyl, pyrimidinediyl or 1,3,5-triazinediyl, each of which is optionally mono- or disubstituted by identical or different substituents selected from the group consisting of fluorine, chlorine, cyano, methyl, cyclopropyl, methoxy, methylthio, trifluoromethyl, difluoromethoxy, trifluoromethoxy, difluorochloromethoxy,
R¹ represents methoxy or methylamino,
R² represents cyano, thiocyanato, aminocarbonyl, aminothiocarbonyl, acetyl, propionyl, pivaloyl, methoxycarbonyl, ethoxycarbonyl, methylaminocarbonyl, ethylaminocarbonyl or dimethylaminocarbonyl,
T represents a single bond, represents oxygen, sulphur, -CH₂-O-, CH₂-S-, methylene, ethylene or propylene, and
Z represents phenyl, pyridyl, pyrimidyl or thienyl, each of which is optionally mono- to trisubstituted by identical or different substituents selected from the group consisting of fluorine, chlorine, bromine, cyano, methyl, ethyl, n- or i-propyl, n-, i-, s- or t-butyl, trifluoromethyl, methoxy, ethoxy, n- or i-propoxy, difluoromethoxy, difluorochloromethoxy, trifluoroethoxy, trifluoromethoxy and methylenedioxy or ethylenedioxy, each of which is doubly attached and each of which is optionally mono- to tetrasubstituted by identical or different substituents selected from the group consisting of fluorine, chlorine, methyl, trifluoromethyl and ethyl.

7. Pesticides, characterized in that they comprise at least one compound of the formula (I) according to Claim 1.

8. Method for controlling pests, characterized in that compounds of the formula (I) according to Claim 1 are allowed to act on pests and/or their habitat.

9. Process for preparing compounds of the formula (I) according to Claim 1, characterized in that
a) hydroxyl compounds of the general formula (II) 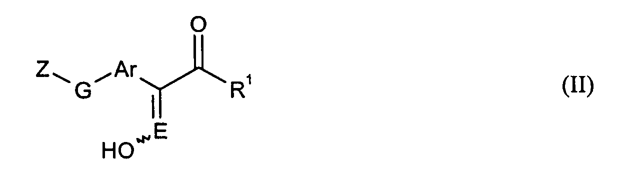 in which
Ar, E, G, R¹ and Z are each as defined in Claim 1
are reacted with a halogen compound of the general formula (III)
X¹-CH₂-R² (III)
in which
X¹ represents halogen and
R² is as defined above,
if appropriate in the presence of an acid acceptor and if appropriate in the presence of a diluent; or when
b) hydroxyaryl compounds of the general formula (IV) 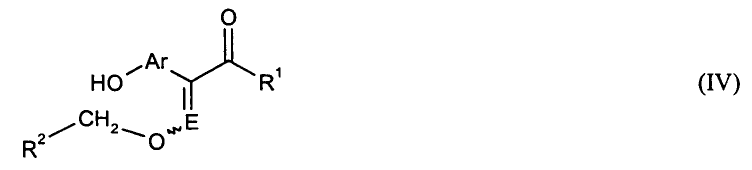 in which
Ar, E, R¹ and R² are each as defined in Claim 1,
are reacted with an aryl compound of the general formula (V) 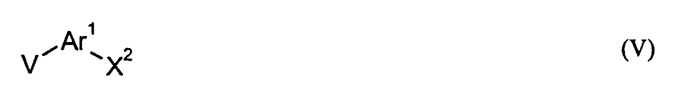 in which
Ar¹ is as defined in Claim 1,
V represents halogen or Z-T-, where
Z and T are each as defined in Claim 1, and
X² represents halogen, alkylsulphonyl or arylsulphonyl,
if appropriate in the presence of an acid acceptor and if appropriate in the presence of a diluent.

10. Use of compounds of the formula (I) according to Claims 1 to 6 for controlling pests.

11. Process for preparing pesticides, characterized in that compounds of the formula (I) according to Claims 1 to 6 are mixed with extenders and/or surfactants.

12. Compounds of the formula (IV) 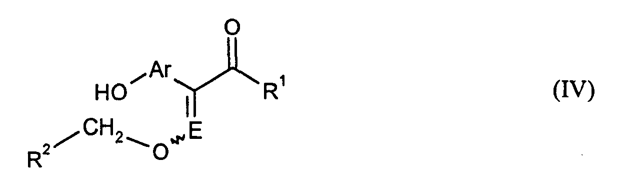 in which
Ar, E, R¹ and R² are each as defined in Claim 1.

13. Compounds of the formula (VI) 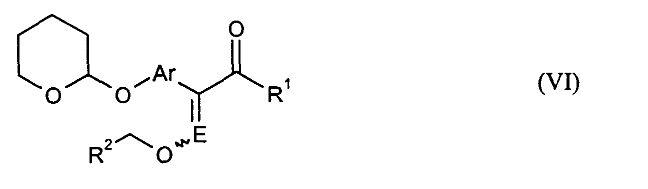 in which
Ar, E, R¹ and R² are each as defined in Claim 1.

## Revendications

1. Composés de formule (I) 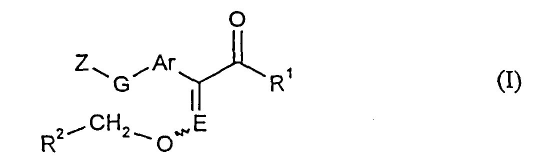 dans laquelle
Ar représente un groupe arylène ou hétéroarylène éventuellement substitué dans chaque cas,
E est un groupe =CH- ou l'azote,
G représente une liaison simple, l'oxygène, le soufre, ou un groupe alcanediyle, alcènediyle, alcynediyle portant chacun le cas échéant un substituant halogéno, hydroxy, alkyle, halogénalkyle ou cyloalkyle, ou l'un des groupements suivants :
-Q-CQ-, -CQ-Q-, -CH₂-Q-; -Q-CH₂-, -CQ-Q-CH₂-, -CH₂-Q-CQ-,-Q-CQ-CH₂-,-Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- ou -T-Ar¹-Q-,
dans lesquels
Ar¹ est un groupe arylène, hétéroarylène, cycloalkylène ou hétérocycloalkylène éventuellement substitué,
n représente les nombres 0, 1 ou 2,
Q est l'oxygène ou le soufre,
R³ est l'hydrogène, un groupe cyano ou un groupe alkyle, alkoxy, alkylthio, alkylamino, dialkylamino ou cycloalkyle, chacun étant éventuellement substitué, et
R⁴ représente l'hydrogène, un groupe hydroxy, cyano, ou un groupe alkyle, alkoxy ou cycloalkyle dont chacun est éventuellement substitué, et
R⁵ représente l'hydrogène ou un groupe alkyle, et
T représente une liaison simple, l'oxygène, le soufre, un groupe -CH₂-O-, -CH₂-S- ou un groupe alcanediyle éventuellement substitué,
R¹ est un groupe alkoxy, alkylamino ou dialkylamino,
R² est un groupe cyano, thiocyanato, alkylcarbonyle, alkoxycarbonyle, aminocarbonyle, aminothiocarbonyle, alkylaminocarbonyle ou dialkylaminocarbonyle, et
Z représente un groupe alkyle, alcényle, alcynyle, cycloalkyle, aryle ou hétérocyclyle dont chacun est éventuellement substitué.

2. Composés de formule (I) suivant la revendication 1, dans laquelle
Ar représente un groupe phénylène ou naphtylène dont chacun est éventuellement substitué, un groupe hétéroarylène monocyclique ou bicyclique à noyau de 5 ou 6 atomes dans chaque cas ou un groupe hétéroarylène condensé au benzène à noyau de 5 ou 6 atomes dont au moins l'un dans chaque cas est un atome d'oxygène, de soufre ou d'azote et le cas échéant un ou deux autres représentent de l'azote, les substituants possibles étant choisis parmi ceux qui sont énumérés ci-après : halogène, cyano, nitro, amino, hydroxy, formyle, carboxy, carbamoyle, thiocarbamoyle, groupe alkyle, alkoxy, alkylthio, alkylsulfinyle ou alkylsulfonyle linéaire ou ramifié dans chaque cas ayant chacun 1 à 6 atomes de carbone, groupe alcényle, alcényloxy ou alcynyloxy linéaire ou ramifié dans chaque cas ayant chacun 2 à 6 atomes de carbone, groupe halogénalkyle, halogénalkoxy, halogénalkylthio, halogénalkylsulfinyle ou halogénalkylsulfonyle linéaire ou ramifié dans chaque cas avec chacun 1 à 6 atomes de carbone et 1 à 13 atomes d'halogènes identiques ou différents, groupe halogénalcényle ou halogénalcényloxy linéaire ou ramifié dans chaque cas avec chacun 2 à 6 atomes de carbone et 1 à 11 atomes d'halogènes identiques ou différents, groupe alkylamino, dialkylamino, alkylcarbonyle, alkylcarbonyloxy, alkoxycarbonyle, alkylsulfonyloxy, hydroximinoalkyle ou alkoximinoalkyle linéaire ou ramifié dans chaque cas ayant chacun 1 à 6 atomes de carbone dans les parties alkyle individuelles, groupe alkylène ou dioxyalkylène à deux liaisons, ayant chacun 1 à 6 atomes de carbone, chacun étant substitué le cas échéant une ou plusieurs fois identiques ou différentes par un halogène et/ou un radical alkyle linéaire ou ramifié ayant 1 à 4 atomes de carbone et/ou un radical halogénalkyle linéaire ou ramifié ayant 1 à 4 atomes de carbone et 1 à 9 atomes d'halogènes identiques ou différents,
E est un groupe =CH- ou l'azote,
G représente une liaison simple, l'oxygène, le soufre ou un groupe alcanediyle, alcènediyle, alcynediyle ayant chacun jusqu'à 4 atomes de carbone et chacun étant substitué par un radical halogéno, hydroxy, alkylé en C₁ à C₄, halogénalkyle en C₁ à C₄ ou cycloalkyle en C₃ à C₆, ou l'un des groupements suivants :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -(O)ₙ-CH₂-, -C(R³)=N-O-, -C(R³)=N-CH₂-, -N(R⁴), -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- ou -T-Ar¹-Q-, où
n représente les nombres 0, 1 ou 2,
Q est l'oxygène ou le soufre,
R³ est l'hydrogène, un groupe cyano, un groupe alkyle, alkoxy, alkylthio, alkylamino ou dialkylamino ayant chacun 1 à 6 atomes de carbone dans les groupes alkyle et portant chacun le cas échéant un substituant halogéno, cyano ou alkoxy en C₁ à C₁ ou un groupe cycloalkyle de 3 à 6 atomes de carbone éventuellement substitué par un radical halogéno, cyano, carboxy, alkyle en C₁ à C₁ ou (alkoxy en C₁ à C₁)-carbonyle, et
R⁴ représente l'hydrogène, un groupe hydroxy, cyano ou un groupe alkyle ayant 1 à 6 atomes de carbone éventuellement substitué par un radical halogéno, cyano ou alkoxy en C₁ à C₁, ou un groupe cycloalkyle de 3 à 6 atomes de carbone éventuellement substitué par un radical halogéno, cyano, carboxy, alkyle en C₁ à C₄ ou (alkoxy en C₁ à C₄)-carbonyle, et
R⁵ représente l'hydrogène ou un groupe alkyle ayant 1 à 4 atomes de carbone,
Ar¹ est un groupe phénylène, naphtylène, cycloalkylène portant chacun le cas échéant un ou plusieurs substituants identiques ou différents ou un groupe hétéroarylène ou hétérocycloalkylène à noyau de 3 à 7 atomes dont au moins l'un est un étome d'oxygène, de soufre ou d'azote et le cas échéant un ou deux autres représentent de l'azote, les substituants possibles étant choisis parmi les suivants :
halogéno, cyano, nitro, amino, hydroxy, formyle, carboxy, carbamoyle, thiocarbamoyle ;
groupe alkyle, alkoxy, alkylthio, alkyl-sulfinyle ou alkylsulfonyle étant chacun linéaire ou ramifié et ayant chacun 1 à 6 atomes de carbone ;
groupe alcényle ou alcényloxy étant chacun linéaire ou ramifié et ayant chacun 2 à 6 atomes de carbone ;
groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents ; groupe halogénalcényle ou halogénalcényloxy étant chacun linéaire ou ramifié et ayant chacun 2 à 6 atomes de carbone et 1 à 11 atomes d'halogènes identiques ou différents ;
groupe alkylamino, dialkylamino, alkylcarbonyle, alkylcarbonyloxy, alkoxycarbonyle, alkylsulfonyloxy, hydroximinoalkyle ou alkoximinoalkyle étant chacun linéaire ou ramifié et ayant chacun 1 à 6 atomes de carbone dans les parties alkyle individuelles, ainsi que
groupe cycloalkyle ayant 3 à 6 atomes de carbone, et
T représente une liaison simple, l'oxygène, le soufre, un groupe -CH₂-O-, -CH₂-S- ou un groupe alcanediyle ayant 1 à 3 atomes de carbone,
R¹ représente un groupe alkyle, alkoxy, alkylamino ou dialkylamino ayant 1 à 4 atomes de carbone dans les chaînes alkyliques individuelles,
R² représente un groupe cyano, thiocyanato, aminocarbonyle, aminothiocarbonyle ou un groupe alkylcarbonyle, alkoxycarbonyle, alkylaminocarbonyle ou dialkylaminocarbonyle ayant 1 à 4 atomes de carbone dans les chaînes alkyliques individuelles,
Z représente un groupe alkyle ayant 1 à 8 atomes de carbone éventuellement substitué une ou plusieurs fois identiques ou différentes par un radical halogéno, cyano, hydroxy, amino, alkoxy en C₁ à C₄, alkylthio en C₁ à C₄, alkylsulfinyle en C₁ à C₄ ou alkylsulfonyle en C₁ à C₄ (pouvant chacun être substitués le cas échéant par un halogène) ;
un groupe alcényle ou alcynyle ayant chacun jusqu'à 8 atomes de carbone et portant chacun un substituant halogéno ;
un groupe cycloalkyle ayant 3 à 6 atomes de carbone éventuellement substitué dans chaque cas une ou plusieurs fois identiques ou différentes par un radical halogéno, cyano, carboxy, phényle (qui est substitué le cas échéant par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle;
un groupe phényle ou naphtyle portant chacun le cas échéant un ou plusieurs substituants identiques ou différents ou un groupe hétérocyclyle à noyau de 3 à 7 atomes dont au moins l'un est un atome d'oxygène, de soufre ou d'azote et le cas échéant un ou deux autres représentent de l'azote, les substituants possibles étant choisis parmi les suivants :
halogéno, cyano, nitro, amino, hydroxy, formyle, carboxy, carbamoyle, thiocarbamoyle ;
un groupe alkyle, alkoxy, alkylthio, alkylsulfinyle ou alkylsulfonyle étant chacun linéaire ou ramifié et ayant chacun 1 à 6 atomes de carbone ;
un groupe alcényle ou alcényloxy étant chacun linéaire ou ramifié et ayant chacun 2 à 6 atomes de carbone ;
un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents ;
un groupe halogénalcényle ou halogénalcényloxy linéaire ou ramifié ayant dans chaque cas 2 à 6 atomes de carbone et 1 à 11 atomes d'halogènes identiques ou différents ;
un groupe alkylamino, dialkylamino, alkylcarbonyle, alkylcarbonyloxy, alkoxycarbonyle ou alkylsulfonyloxy étant chacun linéaire ou ramifié et ayant chacun 1 à 6 atomes de carbone dans les parties alkyle individuelles ;
un groupe alkylène ou dioxyalkylène ayant chacun 1 à 6 atomes de carbone, chacun deux liaisons, et étant chacun substitué le cas échéant une ou plusieurs fois identiques ou différentes par un radical halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
un groupe cycloalkyle de 3 à 6 atomes de carbone ;
un groupe hétérocyclyle ou hétérocyclyl-méthyle ayant chacun un noyau de 3 à 7 atomes dont chaque fois 1 à 3 hétéroatomes identiques ou différents sont en particulier de l'azote, de l'oxygène et/ou du soufre, ou un groupement 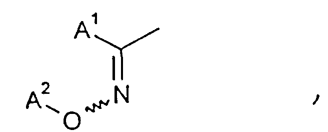 dans lequel
A¹ est un groupe alkyle ayant 1 à 4 atomes de carbone ou cycloalkyle ayant 1 à 6 atomes de carbone et
A² représente un groupe alkyle ayant 1 à 4 atomes de carbone, alcényle ou alcynyle ayant chacun 2 à 4 atomes de carbone, portant le cas échéant un substituant cyano, alkoxy, alkylthio, alkylamino, dialkylamino ou phényle.

3. Composés de formule (I) suivant la revendication 1, dans laquelle
Ar représente un groupe ortho-, méta- ou para-phénylène, furannediyle, thiophènediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle (notamment pyridine-2,3-diyle), pyrimidinediyle, pyridazinediyle, pyrazinediyle, 1,3,4-triazinediyle ou 1,2,3-triazine-diyle portant chacun le cas échéant 1 ou 2 substituants fluoro, chloro, cyano, méthyle, éthyle, cyclopropyle, trifluorométhyle, méthoxy, éthoxy, méthylthio, méthylsulfinyle ou méthylsulfonyle,
E représente en particulier le groupe =CH- ou l'azote,
G représente l'oxygène ou un groupe diméthylène (éthane-1,2-diyle), éthène-1,2-diyle, portant chacun le cas échéant un substituant fluoro, bromo ou chloro, ou l'un des groupements suivants :
-Q-CQ-, -CQ-Q-, -CH₂-Q-; -Q-CH₂-, -CQ-Q-CH₂-, -CH₂-Q-CQ--Q-CQ-CH₂-, -Q-CQ-Q-CH₂-, -N=N-, -S(O)ₙ-, -CH₂-S(O)ₙ-, -CQ-, -S(O)ₙ-CH₂-, -C(R³)=N-O-, -C(R³)=N-O-CH₂-, -N(R⁴)-, -CQ-N(R⁴)-, -N(R⁴)-CQ-, -Q-CQ-N(R⁴)-, -N=C(R³)-Q-CH₂-, -CH₂-O-N=C(R³)-, -C(CH₃)-O-N=C(R³)-, -N(R⁴)-CQ-Q-, -CQ-N(R⁴)-CQ-Q-, -N(R⁴)-CQ-Q-CH₂-, -Q-C(R³)=N-O-CH₂-, -N(R⁴)-C(R³)=N-O-CH₂-, -O-CH₂-C(R³)=N-O-CH₂-, -N=N-C(R³)=N-O-CH₂-, -C(=N-O-R⁵)-C(R³)=N-OCH₂-, -C(=N-O-R⁵)-C(R³)-O-N=CH-, -C(=N-O-R⁵)-C(R³)-O-N=C(CH₃)-, -T-Ar¹- ou -T-Ar¹-Q-,
dans lesquels
n représente les nombres 0, 1 ou 2,
Q est l'oxygène ou le soufre,
R³ est l'hydrogène, un groupe cyano, méthyle, éthyle ou cyclopropyle et
R⁴ est l'hydrogène, un groupe méthyle, éthyle ou cyclopropyle,
R⁵ est l'hydrogène ou un groupe alkyle ayant 1 à 4 atomes de carbone,
Ar¹ est un groupe phénylène ou pyridinediyle portant chacun le cas échéant 1 à 3 substituants identiques ou différents, un groupe pyrimidinediyle, pyridazinediyle, pyrazinediyle, 1,2,3-triazinediyle, 1,2,4-triazinediyle ou 1,3,5-triazinediyle portant chacun le cas échéant un seul substituant ou un groupe 1,2,4-thiadiazolediyle, 1,3,4-thiadiazolediyle, 1,2,4-oxadiazolediyle, 1,3,4-oxadiazolediyle, les substituants possibles étant choisis de préférence parmi les suivants :
fluor, chlore, brome, cyano, méthyle, éthyle, n-propyle, isopropyle, cyclopropyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle et
T est une liaison simple, l'oxygène, le soufre, un groupe -CH₂-O-, -CH₂-S-, méthylène, éthylène ou propylène,
R¹ est un groupe méthyle, éthyle, n-propyle, isopropyle, méthoxy, éthoxy, n-propoxy, isopropoxy, méthylamino, éthylamino, n-propylamino, isopropylamino, diméthylamino, diéthylamino ou N-méthyl-N-éthylamino,
R² est un groupe cyano, thiocyanato, aminocarbonyle, aminothiocarbonyle, acétyle, propionyle, pivaloyle, méthoxycarbonyle, éthoxycarbonyle, méthyl-amino-carbonyle, éthylaminocarbonyle ou diméthylamino-carbonyle,
Z représente un groupe phényle, 1,2,4-thiadiazolyle, 1,3,4-thiadiazolyle, 1,2,4-oxadiazolyle, 1,3,4-oxadiazolyle, pyridinyle, pyrimidyle, pyridazinyle, pyrazinyle, 1,2,3-triazinyle, 1,2,4-triazinyle ou 1,3,5-triazinyle portant chacun le cas échéant 1 à 3 substituants identiques ou différents, les substituants possibles étant choisis parmi les suivants :
fluoro, chloro, bromo, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluoro-chlorométhoxy, trifluoréthoxy, difluorométhylthio, trifluorométhylthio, difluorochlorométhylthio, tri-fluorométhylsulfinyle ou trifluorométhylsulfonyle, méthoxycarbonyle, éthoxycarbonyle,
un groupe méthylènedioxy ou éthylènedioxy portant chacun deux liaisons et chacun étant éventuellement substitué 1 à 4 fois identiques ou différentes par un radical fluoro, chloro, méthyle, trifluorométhyle ou éthyle,
ou bien un groupement 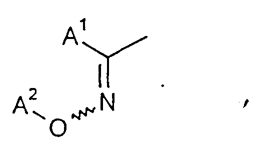 dans lequel
A¹ représente un groupe méthyle, éthyle, n-propyle, isopropyle, n-butyle, isobutyle, sec.-butyle, tertio-butyle, cyclopropyle ou cyclobutyle,
A² représente un groupe méthyle, éthyle, n-propyle, isopropyle, n-butyle, isobutyle, sec.-butyle, tertio-butyle, allyle, propargyle, but-2-ène-1-yle, 2-méthyl-prop-1-ène-3-yle, cyanométhyle, méthoxyméthyle, éthoxyméthyle, méthoxyéthyle, éthoxyéthyle, méthylthiométhyle, éthylthio-méthyle, méthylthioéthyle, éthylthioéthyle, di-méthylaminométhyle, diméthylaminoéthyle, méthyl-aminométhyle, méthylaminoéthyle ou benzyle.

4. Composés de formule (I) suivant la revendication 1, dans laquelle
Ar est un groupe ortho-phénylène, pyridine-2,3-diyle ou thiophène-2,3-diyle,
E est le groupe =CH- ou l'azote,
G est un groupe -O-CH₂-,
R¹ est un groupe méthoxy ou méthylamino,
R² est un groupe cyano, thiocyanato, aminocarbonyle, aminothiocarbonyle, acétyle, propionyle, pivaloyle, méthoxycarbonyle, éthoxycarbonyle, méthylaminocarbonyle, éthylaminocarbonyle ou diméthylaminocarbonyle et
Z est un groupe phényle portant éventuellement dans chaque cas 1 à 3 substituants identiques ou différents, les substituants possibles étant choisis de préférence parmi les suivants :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle éthylsulfinyle, méthylsulfonyle, éthylsulfonyle, trifluorométhyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxy-carbonyle,
un groupe méthylènedioxy ou éthylènedioxy ayant chacun deux liaisons et chacun étant substitué le cas échéant 1 à 4 fois identiques ou différentes par du fluor, du chlore, un radical méthyle, trifluorométhyle ou éthyle
ou un groupement 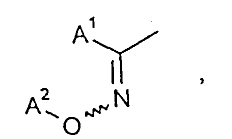 dans lequel
A¹ est un groupe méthyle, éthyle, n-propyle, isopropyle, n-butyle, isobutyle, sec.-butyle, tertio-butyle, cyclopropyle ou cyclobutyle, et
A² est un groupe méthyle, éthyle, n-propyle, isopropyle, n-butyle, isobutyle, sec.-butyle, tertiobutyle, allyle, propargyle, but-2-ène-1-yle, 2-méthyl-prop-1-ène-3-yle, cyanométhyle, méthoxyméthyle, éthoxyméthyle, méthoxyéthyle, éthoxyéthyle, méthylthiométhyle, éthyl-thiométhyle, méthylthioéthyle, éthylthioéthyle, diméthylaminométhyle, diméthylaminoéthyle, méthylaminométhyle, méthylaminoéthyle ou benzyle.

5. Composés de formule (I) suivant la revendication 1, dans laquelle
Ar est un groupe ortho-phénylène, pyridine-2,3-diyle ou thiophène-2,3-diyle,
E est le groupe =CH- ou l'azote,
G est un groupe -C(R³)=N-O-CH₂-,
R³ est un groupe méthyle ou cyclopropyle,
R¹ est un groupe méthoxy ou méthylamino,
R² est un groupe cyano, thiocyanato, aminocarbonyle, aminothiocarbonyle, acétyle, propionyle, pivaloyle, méthoxycarbonyle, éthoxycarbonyle, méthyl-amino-carbonyle, éthylaminocarbonyle ou diméthylamino-carbonyle, et
Z est un groupe phényle, pyridyle ou pyrimidyle portant éventuellement dans chaque cas 1 à 3 substituants identiques ou différents, les substituants possibles étant choisis de préférence parmi les suivants :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthyl-sulfonyle, éthylsulfonyle, trifluorométhyle, trifluoréthyle, difluorométhoxy, trifluorométhoxy, difluoro-chlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle, méthoximino-méthyle, éthoximinométhyle, méthoximinoéthyle, éthoximino-éthyle,
un groupe méthylènedioxy ou éthylènedioxy ayant dans chaque cas deux liaisons et portant chacun le cas échéant 1 à 4 substituants fluoro, chloro, méthyle, trifluorométhyle, ou éthyle identiques ou différents.

6. Composés de formule (I) suivant la revendication 1,
dans laquelle
Ar est un groupe ortho-phénylène,
E est le groupe =CH- ou l'azote,
G est l'oxygène ou un groupe -T-Ar¹-O-,
Ar¹ est un groupe 1,2,4-thiadiazolediyle, 1,3,4-thiadiazolediyle, 1,2,4-oxadiazolediyle, 1,3,4-oxadiazolediyle ou un groupe pyridinediyle, pyrimidinediyle ou 1,3,5-triazinediyle, chacun portant le cas échéant un ou deux substituants, identiques ou différents, fluoro, chloro, cyano, méthyle, cyclopropyle, méthoxy, méthylthio, trifluorométhyle, difluorométhoxy, trifluorométhoxy, difluorochlorométhoxy,
R¹ est un groupe méthoxy ou méthylamino,
R² est un groupe cyano, thiocyanato, aminocarbonyle, aminothiocarbonyle, acétyle, propionyle, pivaloyle, méthoxycarbonyle, éthoxycarbonyle, méthylaminocarbonyle, éthylaminocarbonyle ou diméthylaminocarbonyle,
T est une liaison simple, l'oxygène, le soufre, un groupe -CH₂-O-, CH₂-S-, méthylène, éthylène ou propylène et
Z est un groupe phényle, pyridyle, pyrimidyle ou thiényle portant chacun le cas échéant 1 à 3 substituants, identiques ou différents, fluoro, chloro, bromo, cyano, méthyle, éthyle, n-propyle, isopropyle, n-butyle, iso-butyle, sec.-butyle, tertiobutyle, trifluorométhyle, méthoxy, méthoxy, éthoxy, n-propoxy, isopropoxy, difluorométhoxy, difluorochlorométhoxy, trifluoréthoxy, trifluorométhoxy ou un groupe méthylènedioxy ou éthylènedioxy ayant chacun deux liaisons et chacun portant le cas échéant 1 à 4 substituants, identiques ou différents, fluoro, chloro, méthyle, trifluorométhyle ou éthyle.

7. Compositions pesticides, caractérisées par une teneur en au moins un composé de formule (I) suivant la revendication 1.

8. Procédé pour combattre des parasites, caractérisé en ce qu'on fait agir des composés de formule (I) suivant la revendication 1 sur les parasites et/ou sur leur milieu.

9. Procédé de production de composés de formule (I) suivant la revendication 1, caractérisé en ce que
a) on fait réagir des composés hydroxylés de formule générale (II) 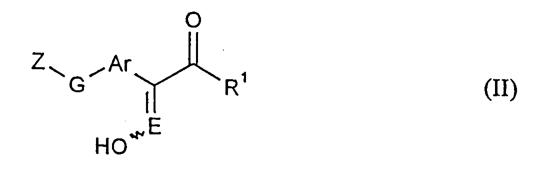 dans laquelle
Ar, E, G, R¹ et Z ont les définitions indiquées dans la revendication 1,
avec un composé halogéné de formule générale (III)
X¹-CH₂-R² (III)
dans laquelle
X¹ est un halogène et
R² a la définition indiquée ci-dessus,
le cas échéant en présence d'un accepteur d'acide et en la présence éventuelle d'un diluant ; ou bien
b) on fait réagir des composés hydroxyaryliques de formule générale (IV) 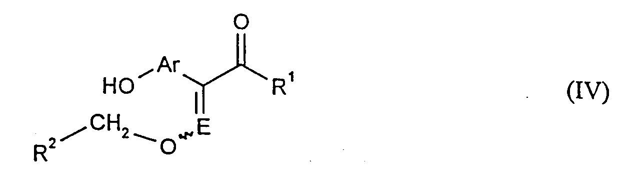 dans laquelle
Ar, E, R¹ et R² ont les définitions indiquées dans la revendication 1
avec un composé arylique de formule générale (V) 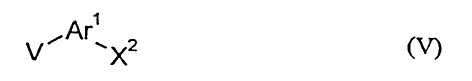 dans laquelle
Ar¹ a la définition indiquée dans la revendication 1,
V est un halogène ou un groupe Z-T-,
Z et T ayant les définitions indiquées dans la revendication 1 et
X² est un halogène, un groupe alkylsulfonyle ou arylsulfonyle,
le cas échéant en présence d'un accepteur d'acide et en la présence éventuelle d'un diluant.

10. Utilisation de composés de formule (I) suivant les revendications 1 à 6 pour combattre des parasites.

11. Procédé de préparation de compositions pesticides, caractérisé en ce qu'on mélange des composés de formule (I) suivant les revendications 1 à 6 avec des diluants et/ou des agents tensio-actifs.

12. Composés de formule (IV) 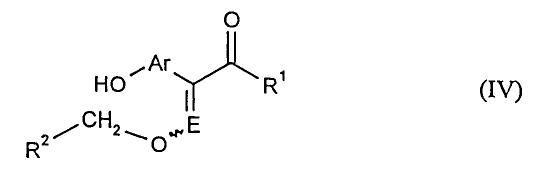 dans laquelle
Ar, E, R¹ et R² ont les définitions indiquées dans la revendication 1.

13. Composés de formule (IV) 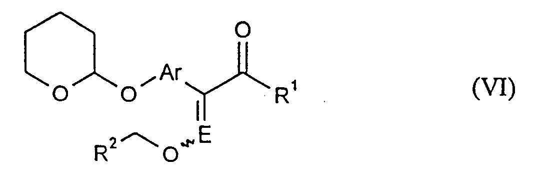 dans laquelle
Ar, E, R¹ et R² ont les définitions indiquées dans la revendication 1.
